(19) **Europäisches Patentamt / European Patent Office / Office européen des brevets**

(11) **EP 3 936 870 B1**

(12) **EUROPEAN PATENT SPECIFICATION**

(45) Date of publication and mention of the grant of the patent:
**24.04.2024 Bulletin 2024/17**

(21) Application number: **20769728.5**

(22) Date of filing: **06.03.2020**

(51) International Patent Classification (IPC):
**G01N 33/68** (2006.01)    **G01N 30/72** (2006.01)
**G01N 30/88** (2006.01)    **G01N 33/574** (2006.01)

(52) Cooperative Patent Classification (CPC):
**G01N 33/6848; G01N 33/57415;** G01N 2333/912

(86) International application number:
**PCT/KR2020/003138**

(87) International publication number:
**WO 2020/184905 (17.09.2020 Gazette 2020/38)**

(54) **METHOD FOR QUANTITATION OF HER2 IN BREAST CANCER SAMPLE BY MASS SPECTROMETRY AND SCORING OF HER2 STATE BY USING SAME**

VERFAHREN ZUR QUANTIFIZIERUNG VON HER2 IN EINER BRUSTKREBSPROBE DURCH MASSENSPEKTROMETRIE UND RITZEN DES HER2 -ZUSTANDES UNTER VERWENDUNG DESSELBEN

MÉTHODE DE QUANTIFICATION DE HER2 DANS UN ÉCHANTILLON DE CANCER DU SEIN PAR SPECTROMÉTRIE DE MASSE ET MÉTHODE DE NOTATION DE L'ÉTAT HER2 L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.03.2019 KR 20190026581**
          **31.07.2019 KR 20190093135**

(43) Date of publication of application:
**12.01.2022 Bulletin 2022/02**

(73) Proprietor: **Seoul National University R&DB Foundation**
**Seoul 08826 (KR)**

(72) Inventors:
• **KIM, Youngsoo**
 **Seoul 03080 (KR)**
• **RYU, Han Suk**
 **Seoul 03080 (KR)**
• **KIM, Hyunsoo**
 **Seoul 03081 (KR)**
• **DO, Misol**
 **Seoul 03080 (KR)**

(74) Representative: **Ter Meer Steinmeister & Partner Patentanwälte mbB**
**Nymphenburger Straße 4**
**80335 München (DE)**

(56) References cited:
**KR-A- 20180 058 118**    **US-A1- 2003 152 935**
**US-A1- 2018 187 239**    **US-A1- 2018 187 239**

• **CARINE STEINER ET AL: "Quantification of HER2 by Targeted Mass Spectrometry in Formalin-Fixed Paraffin-Embedded (FFPE) Breast Cancer Tissues", MOLECULAR & CELLULAR PROTEOMICS, vol. 14, no. 10, 6 July 2015 (2015-07-06), pages 2786-2799, XP055754932, US ISSN: 1535-9476, DOI: 10.1074/mcp.O115.049049**
• **KRIZMAN D ET AL: "PP126 Quantitative protein analysis in FFPE tissue: Application to the tissue microenvironment", EUROPEAN JOURNAL OF CANCER. SUPPLEMENT, PERGAMON, OXFORD, GB, vol. 7, no. 4, 1 October 2009 (2009-10-01) , pages 21-22, XP026692196, ISSN: 1359-6349, DOI: 10.1016/S1359-6349(09)72178-0 [retrieved on 2009-10-01]**

- NUCIFORO PAOLO ET AL: "High HER2 protein levels correlate with increased survival in breast cancer patients treated with anti-HER2 therapy", MOLECULAR ONCOLOGY, vol. 10, no. 1, 15 September 2015 (2015-09-15), pages 138-147, XP029362561, ISSN: 1574-7891, DOI: 10.1016/J.MOLONC.2015.09.002
- DO MISOL ET AL: "Clinical Application of Multiple Reaction Monitoring-Mass Spectrometry to Human Epidermal Growth Factor Receptor 2 Measurements as a Potential Diagnostic Tool for Breast Cancer Therapy", CLINICAL CHEMISTRY, vol. 66, no. 10, 1 October 2020 (2020-10-01), pages 1339-1348, XP055948126, US ISSN: 0009-9147, DOI: 10.1093/clinchem/hvaa178
- Carine Steiner, Jean-Christophe Tille, Jens Lamerz, Sabine Kux Van Geijtenbeek, Thomas A. Mckee, Miro Venturi, Laura Rubbia-Brandt: "Quantification of HER2 by targeted mass spectrometry in formalin-fixed paraffin-embedded (FFPE) breast cancer tissues", Molecular & Cellular Proteomics, vol. 14, no. 10, 2015, pages 2786-2799, XP055754932, DOI: 10.1074/mcp.O115.049049
- Yi Chen, David J. Britton, Elizabeth R. Wood, Anthony M. Magliocco, Ian Pike And John M. Koomen: "Abstract 2494: Liquid chromatography-multiple reaction monitoring mass spectrometry biomarker quantification for breast cancer patient assessment", Cancer Research. Proceedings: AACR 104th Annual Meeting 2013; Apr 6-10, 2013; Washington, DC, April 2013 (2013-04), XP055754942,

Remarks:

The complete document including Reference Table(s) and the Sequence Listing(s) can be downloaded from the EPO website

**Description**

BACKGROUND OF THE INVENTION

**Field of the Invention**

The present disclosure is related to a method of quantifying HER2 protein in breast cancer tissue sample by mass spectrometry and its use in companion diagnostics.

**Description of the Related Art**

Breast cancer ranks second in the female cancer incidence rate in Korea (14.8%), and the breast cancer incidence rate is 52.1 per 100,000 female population, ranking first among Asian countries. According to the statistics of the Review and Assessment Service, the number of breast cancer patients in Korea increased by about 40,000 over the past five years, and the number of breast cancer-related claims and medical care benefit costs increased by 123.2% and 151.4% in 2017 compared to 2013, respectively.

HER2-receptor-positive breast cancer patients account for 15-20% of all breast cancer patients. HER2-receptor-positive breast cancer is more aggressive than other types of breast cancer, and the prognosis is poor due to the high probability of recurrence, and thus the survival rate is low. Therefore, accurately measuring the expression level of HER2 protein in breast cancer patients is very important in determining the prognosis and treatment strategy of HER2 receptor-positive breast cancer patients.

However, there is no universal gold standard that can accurately measure the expression level of HER2 protein so far. Analysis and evaluation of HER2 protein and expression using Immunohistochemistry (IHC) and evaluation of HER2 gene amplification using Fluorescent in situ Hybridization (FISH) are FDA-approved methods and the most important technique that has been used so far to determine HER2-positive and negative breast cancers.

However, the IHC technique cannot quantitatively measure the expression level of HER2 protein, and depends on the pathologist who determines visually the level of immunostaining of the HER2 protein. Thus, the subjectivity of the pathologist may be involved resulting in the false-positive or false-negative determination leading to a low reproducibility of the determination. When it is found to be HER2 2+ (equivocal) through IHC test, the degree of overexpression of HER2 protein is re-evaluated in more detail using FISH, but the FISH technique is difficult and expensive.

Therefore, it is necessary to develop a technology to accurately quantify HER2 protein expression levels.

US Patent Application Publication No. US 2013/0302328 relates to a method for quantifying full-length HER2 and truncated HER

Korean Patent Application Publication No. 2018-0036653 relates to quantification of HER2 protein for optimal cancer treatment. However, the above documents do not provide a method for correcting the differences in the level of HER2 protein expression by patient or patient samples, and the peptide sequence of the epithelial cell-specific protein and the precursor charge status corresponding to the sequence, etc. are not disclosed.

C. Steiner et al., "Quantification of HER2 by Targeted Mass Spectrometry in Formalin-Fixed Paraffin-Embedded (FFPE) Breast Cancer Tissues", MOLECULAR & CELLULAR PROTEOMICS, US, (20150706), vol. 14, no. 10, pages 2786 - 2799 describes a method of measuring an expression level of HER2 protein in vitro in a breast cacner sample (a FPFEE sample) to provide an information for determining HER2 scoring.

SUMMARY OF THE INVENTION

The present disclosure is to provide a method for accurately determining the level of HER2 expression in a breast cancer sample using mass spectrometry, and determining a HER2 score based on the expression level.

In one aspect of the present disclosure, there is provided method of measuring an expression level of HER2 protein *in vitro* in a breast cancer sample as defined in claim 1.

Specific aspects of the invention are defined in claims 2 to 9.

In another aspect of the present disclosure as defined in claim 10, there is provided a method of determining a HER2 score of a breast cancer sample in need thereof using a method according to the present methods disclosed herein, the method comprising the step of correlating the normalized amount of HER2 peptide with a HER2 score: 0, 1+, 2+FISH-, 2+FISH+, or 3+.

Specific aspects of this embodiment are defined in claims 11 to 13.

ADVANTAGEOUS EFFECTS

The method according to the present invention can determine HER2 scores (0, 1+, 2+FISH-, 2+FISH+, 3+) of the breast cancer sample more accurately and reproducibly than conventional methods through accurate quantification of HER2 protein from Formalin-Fixed, Paraffin-Embedded (FFPE) tissue sample of breast cancer. In particular, the present method can distinguish 2+FISH- and 2+FISH+, which so far can only be distinguished by FISH. This makes possible the simple and more accurate breast cancer diagnosis leading to a customized therapy which can extend the lifespan of patients and dramatically reduce overall socio-economic costs.

## BRIEF DESCRIPTION OF THE DRAWINGS

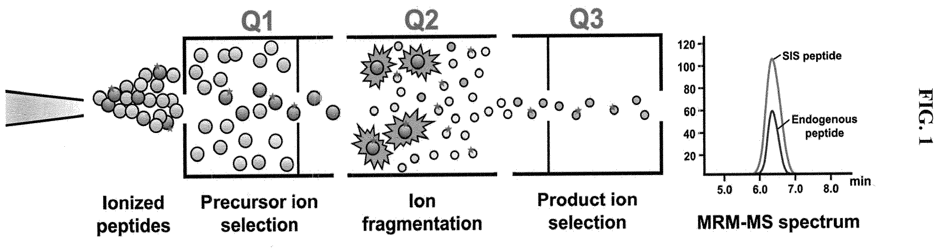

FIG. 1 is a schematic diagram of MRM analysis.

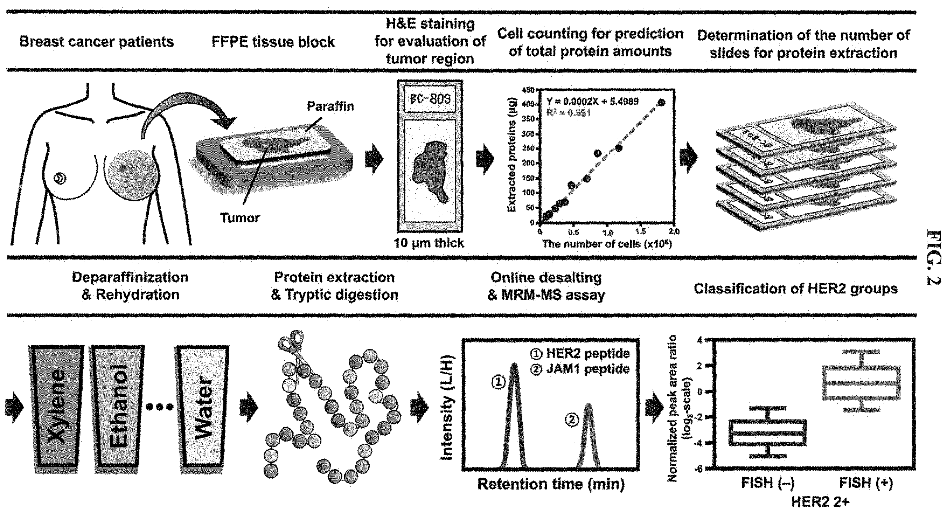

FIG.2 is a schematic diagram of a process of classifying HER2 status through quantification of HER2 and JAM1 peptides used as normalization factor from breast cancer FFPE (Formalin-Fixed Paraffin-Embedded) tissue sections using MRM-MS analysis according to one embodiment of the present disclosure.

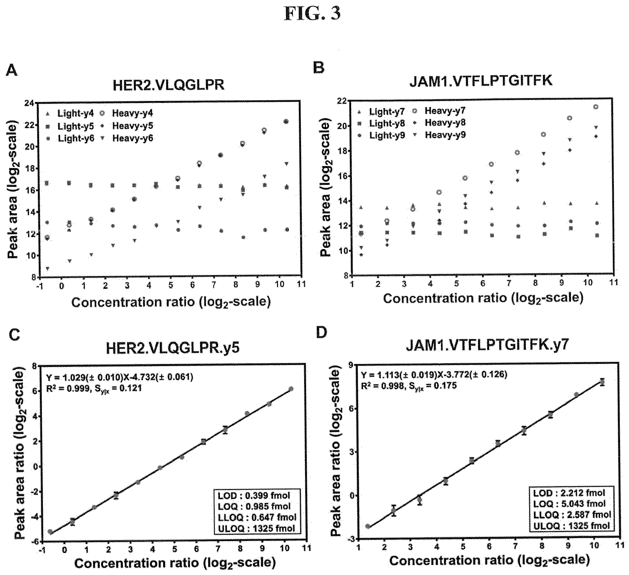

FIG.3 shows a reverse calibration curve for HER2 (VLQGLPR (SEQ ID NO: 1), y5) and JAM1 (VTFLPTGITFK (SEQ ID NO: 3), y7) according to one embodiment of the present disclosure. Product ions with a wide dynamic range were selected through the reverse calibration curve for the target peptides, and the detection and quantitation limits (LOD, LOQ) and other variables (Linearity, LLOQ, ULOQ) for the product ions were determined.

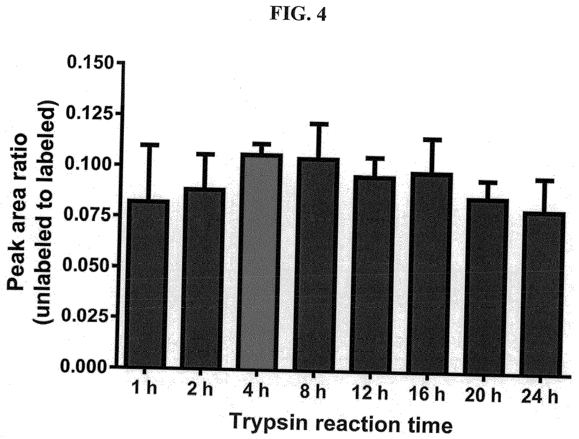

FIG.4 is a graph showing the peak area ratio of the HER2 peptide product ion according to the reaction time with trypsin in one embodiment of the present disclosure. Since the results of MRM-MS analysis of HER2 product ions may differs depending on the reaction time with trypsin, the reaction time that optimizes the peak area ratio of the HER2 product ions was determined. The optimal reaction time with trypsin was determined as 4 hours.

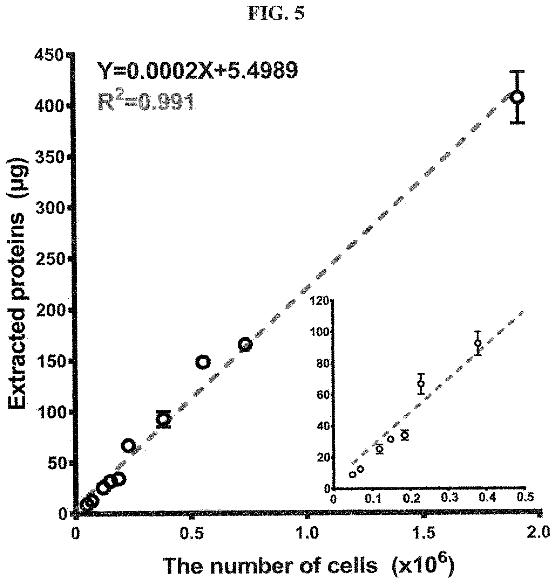

FIG.5 is a standard curve for the amount of protein extracted per cell number in one embodiment of the disclosure. Because the size of the tumor region in a sample varies from sample to sample, the number of FFPE tissue slides that must be used to extract the same amount of protein also varies from sample to sample. In addition, the greater the difference in the amount of protein extracted from each sample, the greater the difference in peptide yield between the samples. Therefore, in order to ensure that the similar or same amount of protein is extracted from different samples, for a sample with a relatively small tumor region, it is necessary to extract proteins from several consecutive tissue sections. In this context, in order to extract a similar amount of protein from each sample, it is necessary to objectively determine how many slides to use for each sample. Here this was solved by using a standard curve representing the correlation between the number of cells present in the tumor region and the amount of protein extracted from the tumor region.

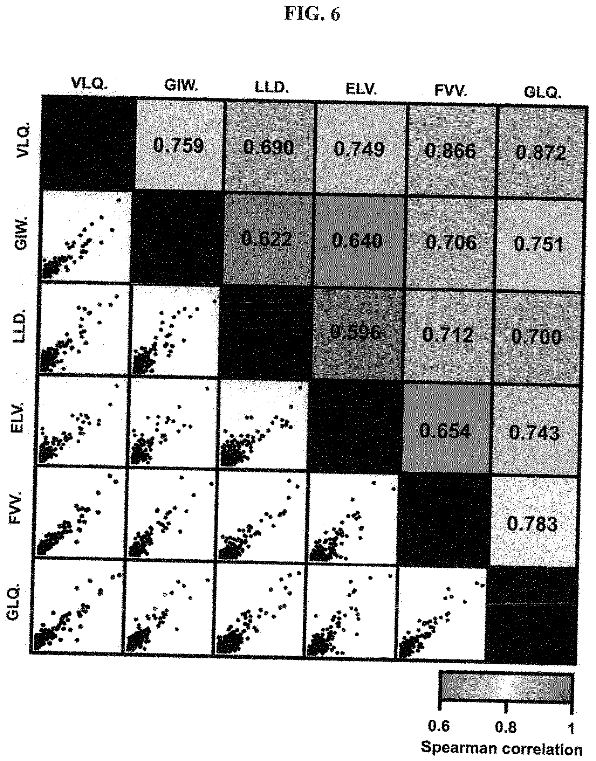

FIG.6 shows Spearman rank correlation-1 between six HER2 peptides according to one embodiment of the present disclosure. After performing MRM-MS analysis using 210 individual samples and the Agilent 6400 series, correlations between the 6 peptides were determined through Spearman rank correlation. As a result, it was confirmed that there was a positive correlation between the 6 HER2 peptides.

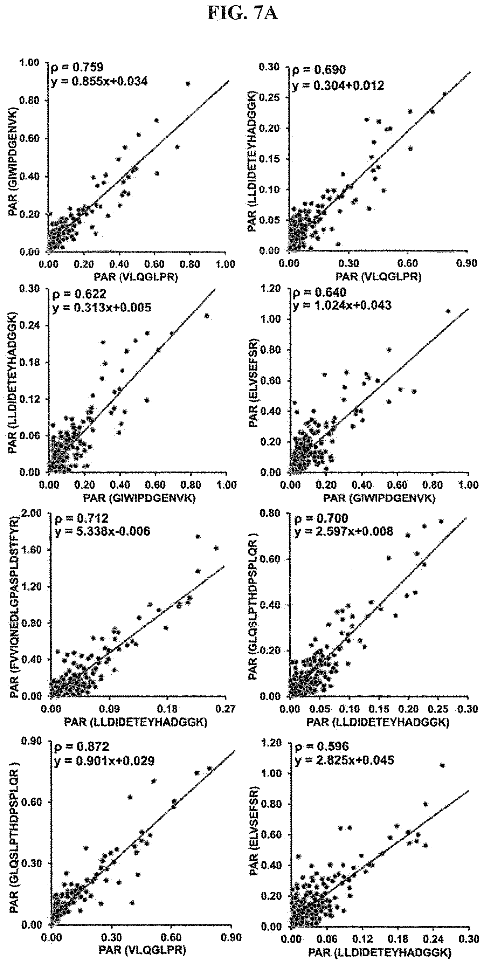

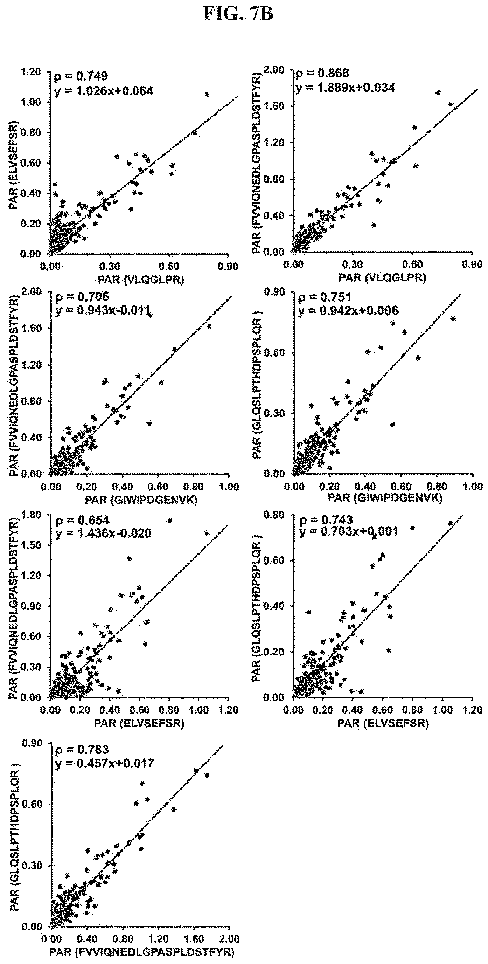

FIG.7 and 7B show Spearman rank correlation-2 between six HER2 peptides according to one embodiment of the present disclosure. After performing MRM-MS analysis using 210 individual samples and the Agilent 6400 series, correlations between the 6 peptides were determined through Spearman rank correlation. As a result, it was confirmed that there was a positive correlation between the 6 HER2 peptides.

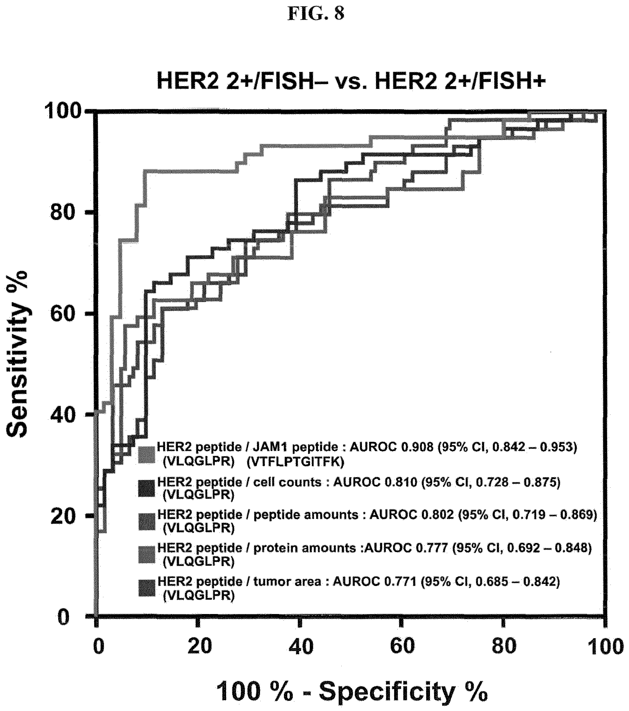

FIG.8 is a ROC curve showing that HER2 2+FISH- group and the HER2 2+FISH+ group can be distinguished when HER2 quantitative analysis values was normalized with the five normalization factors according to one embodiment of the present disclosure. The HER2 quantitative analysis values was divided by the number of cells in the tumor region, the amount of peptide, the amount of protein, the area of the tumor tissue, or the quantitative analysis value of the epithelial cell-specific peptide or housekeeping peptide to select a normalization factor which is best in distinguishing between HER2 2+FISH- group and the HER2 2+FISH+ group. When the HER2 quantitative analysis values were normalized using the quantitative analysis values of the JAM1 peptide among epithelial cell-specific peptides, it was found that the distinction between the two groups was the best with AUC value of 0.908.

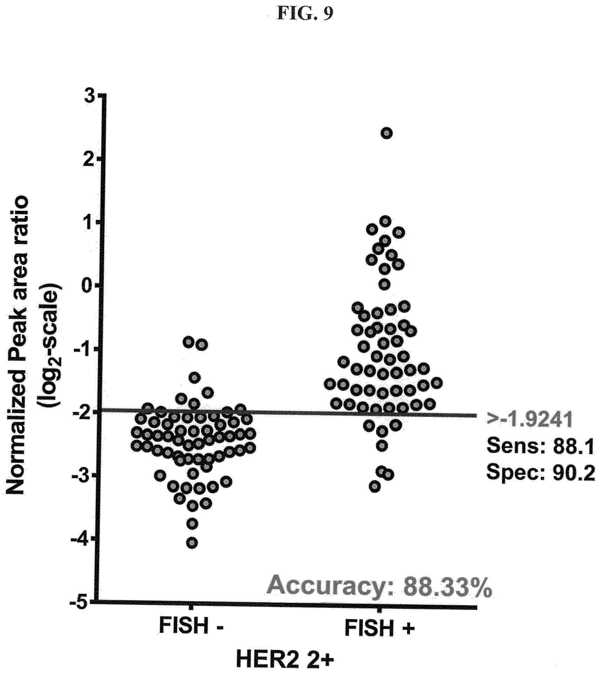

FIG.9 shows the result of distinguishing between HER2 2+FISH- group (61 cases) and the HER2 2+FISH+ group (59 cases) using the HER2 peptide (VLQGLPR (SEQ ID NO: 1)) quantitative analysis value normalized with JAM1 peptide (VTFLPTGITFK (SEQ ID NO: 3)) quantitative analysis value. The peak area ratio of the three product ions (y6, 683.4199+; y5, 570.3358+; y4, 442.2772+) fragmented from the HER2 protein peptide (VLQGLPR (SEQ ID NO: 1), 391.7478++) in the range of collision energy of 3.1 to 23.1 normalized by the peak area ratio of the three product ions (y9, 1023.5873+; y8, 876.5189; y7, 763.4349+) fragmented from the epithelial cell-specific protein JAM1 peptide (VTFLPTGITFK (SEQ ID NO: 3), 612.3554++) in the range of collision energy of 10 to 30 was found to be the best in distinguishing HER2 2+FISH-/+ groups with accuracy of 88.33% and AUC of 0.908. This is a result showing that the two groups that cannot be distinguished by a conventional IHC now can be distinguished by the present MRM-MS analysis.

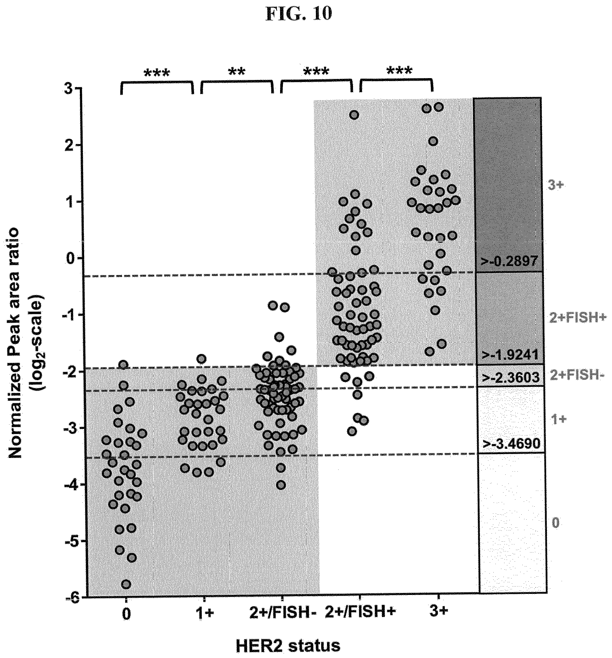

FIG.10 is a result of determining the score over the entire HER2 status using HER2 peptide quantitative analysis values noramlized by JAM1 peptide quantitative analysis values in one embodiment of the present disclosure. For this, the peak area ratio of the three product ions (y6, 683.4199+; y5, 570.3358+; y4, 442.2772+) fragmented from the HER2 protein peptide (VLQGLPR (SEQ ID NO: 1), 391.7478++) in the range of collision energy of 3.1 to 23.1 normalized by the peak area ratio of the three product ions (y9, 1023.5873+; y8, 876.5189; y7, 763.4349+) fragmented from the epithelial cell-specific protein JAM1 peptide (VTFLPTGITFK (SEQ ID NO: 3), 612.3554++) in the range of collision energy of 10 to 30 was used. It was confirmed that all five HER2 status, i.e., HER2 0, 1+, 2+FISH-, 2+FISH+, and 3+, can be distinguished with a significance of p-value < 0.01 or p-value < 0.001. Based on the normalized HER2 peptide quantitative analysis value, the cutoff value required when HER2 scoring is performed was obtained by converting the normalized HER2 peptide quantitative analysis value to a binomial log (log2), and then generating an ROC curve to distinguish adjacent HER2 scores and determining the maximum Youden-index (*J-index*) (*J-index* = sensitivity + specificity - 1). As a result, samples with a binomial logarithmic value of the normalized HER2 peptide quantitative analysis value less than -3.4690 were classified as HER2 0, samples with -3.4690 or more and less than -2.3603 as HER2 1+, samples with -2.3603 or more and less than -1.9241 as HER2 2+FISH-, samples with -1.9241 or more and less than -0.2897 as HER2 2+FISH+, and samples with -0.2897 or more as HER2 3+.

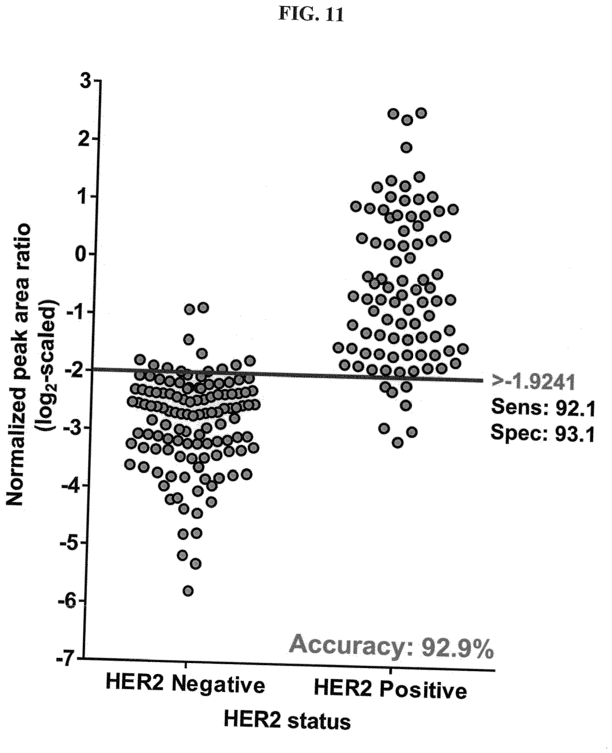

FIG.11 is a result of distinguishing HER2-negative breast cancer group (121 cases) and HER2-positive breast cancer group (89 cases) using HER2 peptide (VLQGLPR (SEQ ID NO: 1)) quantitative analysis result normalized by the quantitative analysis result of JAM1 peptide (VTFLPTGITFK (SEQ ID NO: 3)) according to one embodiment of the present disclosure. For this, the peak area ratio of the three product ions (y6, 683.4199+; y5, 570.3358+; y4, 442.2772+) fragmented from the HER2 protein peptide (VLQGLPR (SEQ ID NO: 1), 391.7478++) in the range of collision energy of 3.1 to 23.1 normalized by the peak area ratio of the three product ions (y9, 1023.5873+; y8, 876.5189; y7, 763.4349+) fragmented from the epithelial cell-specific protein JAM1 peptide (VTFLPTGITFK (SEQ ID NO: 3), 612.3554++) in the range of collision energy of 10 to 30 was used. This is a significant result with accuracy of 92.9% and AUC of 0.960 in distinguishing HER2-negative and positive breast cancer group.

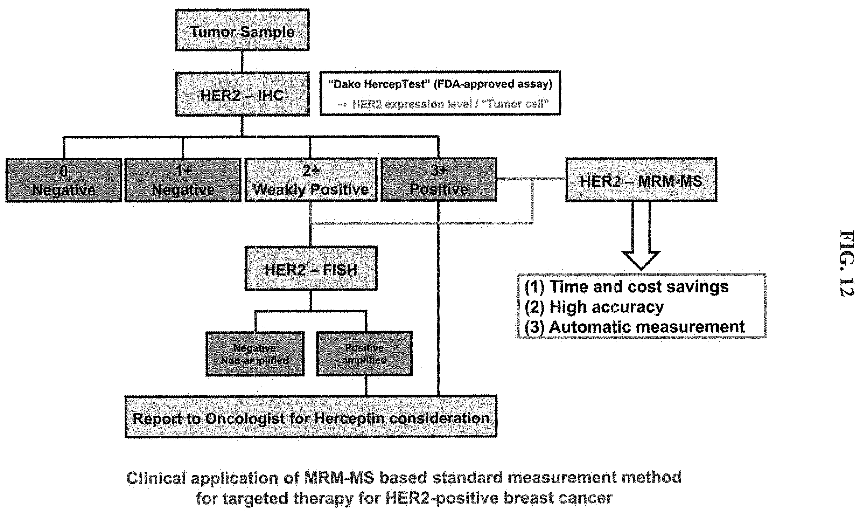

FIG.12 schematically shows a method of classifying HER2 status through MRM-MS analysis according to the present disclosure. There is a difference between the conventional method using IHC and the present method based on MRM-MS analysis in determining HER2 status. The method of the present invention can distinguish the HER2 2+FISH- group and the HER2 2+FISH+ group, which cannot be distinguished by the conventional method IHC, thus significantly reducing the number of equivocal HER2 2+ groups. Further this can lead to saving the cost and time required for FISH by reducing the absolute number of samples that must be performed, and thus it is expected that the overall cost and time required for HER2 status classification can be saved.

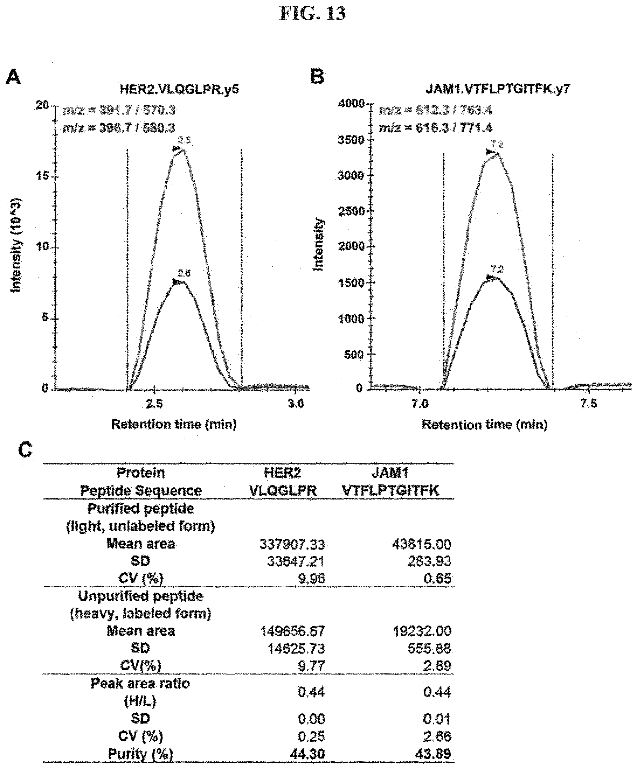

FIG.13 is a result performed to correct the SIS peptide concentration value according to the purity of the SIS peptide which were used for the reverse calibration curve for the VLQGLPR (SEQ ID NO: 1) peptide of the HER2 protein and the VTFLPTGITFK (SEQ ID NO: 3) peptide of the JAM1 protein of FIG. 3. This is to determine the purity of the HER2 (VLQGLPR (SEQ ID NO: 1)) and JAM1 (VTFLPTGITFK (SEQ ID NO: 3)) SIS peptides used in this study. For HER2 (VLQGLPR (SEQ ID NO: 1)) peptide and JAM1 (VTFLPTGITFK (SEQ ID NO: 3)) peptide, MRM-MS assay was performed using equal amounts (200 fmol) of unpurified synthetic peptide (heavy, labeled form) and purified synthetic peptides (light, unlabeled form), respectively. Then the purity of the SIS peptide (unpurified synthetic peptide) used in the reverse calibration curve was calculated:

$$ \text{Purity} = \frac{Peak\ area\ ratio\ of\ unpurified\ synthetic\ peptide}{Peak\ area\ ratio\ of\ purified\ synthetic\ peptide} $$

. The purity of the HER2 (VLQGLPR (SEQ ID NO: 1)) SIS peptide was 44.17%, and the purity of the JAM1 (VTFLPTGITFK (SEQ ID NO: 3)) SIS peptide was 44.15%.

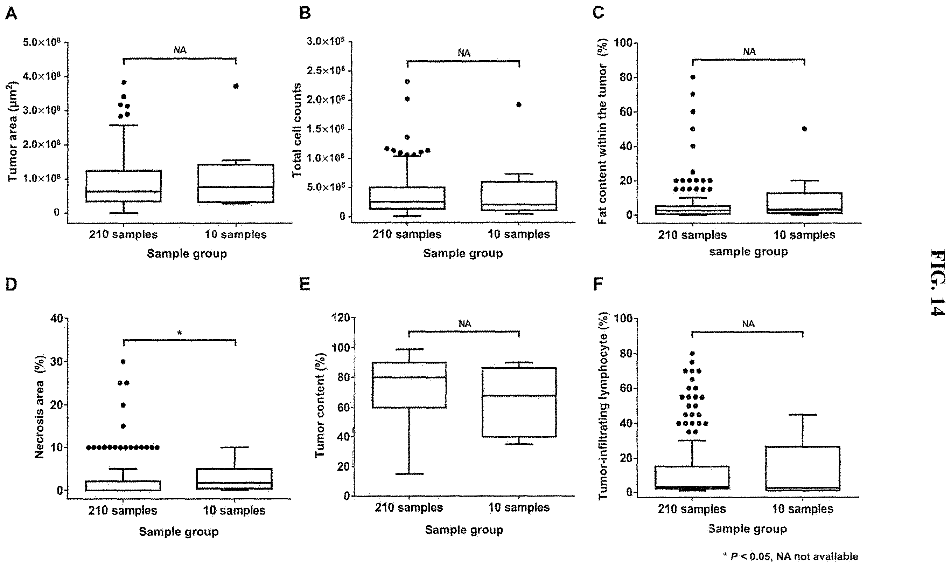

FIG.14 is a result of the experiment to evaluate the representativeness of the 10 samples used in FIG. 5 (standard curve for the amount of protein extracted per cell number) for the entire 210 samples used in this study. There was no statistical difference (P > 0.05) found in 5 histopathological features except for the necrosis area (%) between the 10 samples and 210 samples. As to the necrosis area (%), the difference was also found to be negligible with P-value = 0.045. Therefore, it can be determined that the 10 samples can represent the entire 210 samples, and it can be considered that the 10 samples are suitable for producing a standard curve for the amount of protein extracted per cell number.

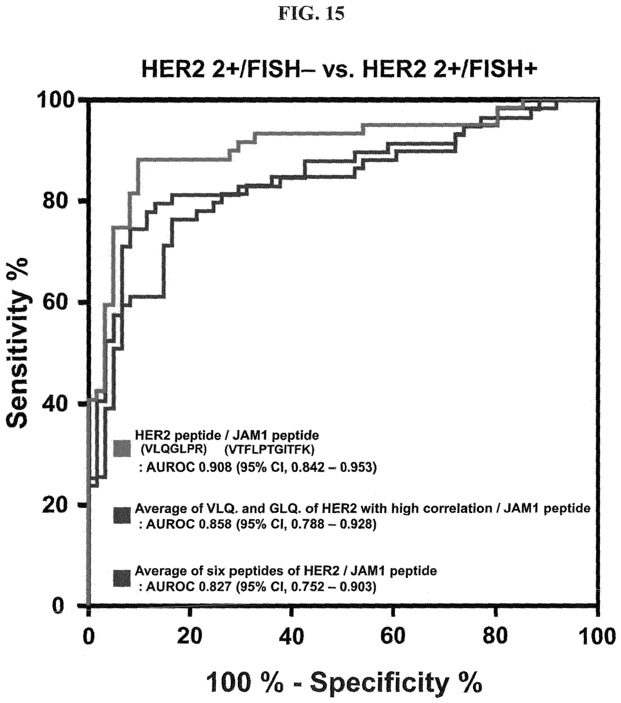

FIG. 15 is a ROC curve showing that the present method can distinguish HER2 2+FISH+ and - groups using the peak area ratio of the HER2 peptide normalized by the peak area ratio of the JAM1 (VTFLPTGITFK (SEQ ID NO: 3)) peptide, which was selected as an optimal normalization factor in one embodiment of the present disclosure. Each of the peak area ratio of a single HER2 peptide (VLQGLPR (SEQ ID NO: 1)), the averaged value of the peak area ratios of the two HER2 peptides (VLQGLPR (SEQ ID NO: 1), GLQSLPTHDPSPLQR (SEQ ID NO: 52) with the highest correlation, and the averaged value of the peak area ratios of the 6 HER2 peptides was normalized by the peak area ratio of the JAM1 (VTFLPTGITFK (SEQ ID NO: 3)) peptide, the value of which was then used for ROC curve generation. It was found that the use of the peak area ratio of a single HER2 peptide (VLQGLPR (SEQ ID NO: 1)) gave the best result in distinguishing HER2 2+FISH+ and - groups with AUC = 0.908 compared to the use of the averaged value of the peak area ratios of several HER2 peptides.

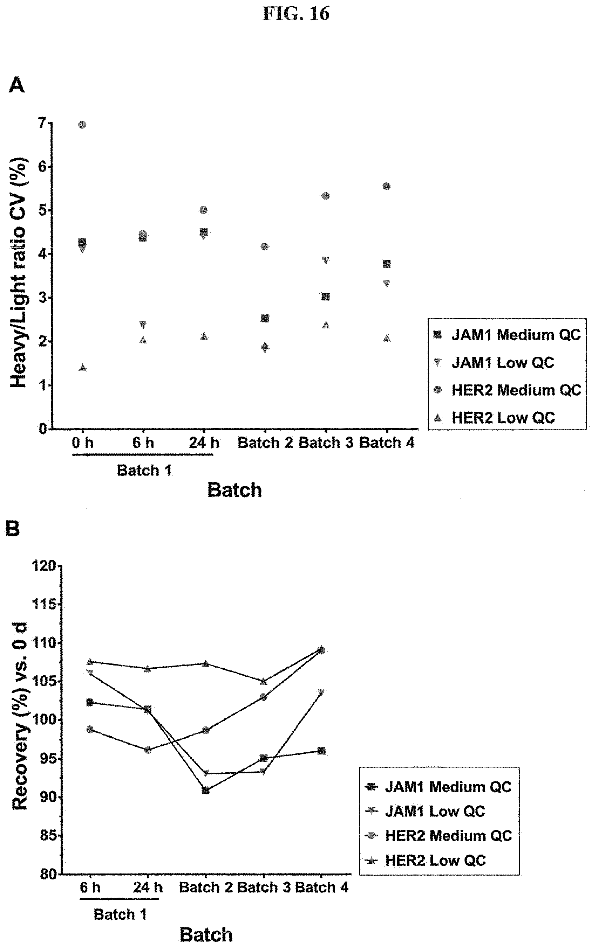

FIG.16 is a result of measuring the stability of the HER2 (VLQGLPR (SEQ ID NO: 1)) peptide and the JAM1 (VTFLPTGITFK (SEQ ID NO: 3)) peptide in accordance with the Clinical Proteomic Tumor Analysis Consortium (CPTAC) guideline. Peak area ratios of HER2 (VLQGLPR (SEQ ID NO: 1)) peptide and JAM1 (VTFLPTGITFK (SEQ ID NO: 3)) peptide were determined in low-quality control (QC) and medium-QC samples under six different conditions. (A) The coefficient of variation (CV) value of the peak area ratios of the HER2 (VLQGLPR (SEQ ID NO: 1)) peptide and the JAM1 (VTFLPTGITFK (SEQ ID NO: 3)) peptide in 6 different conditions in low-QC and medium-QC samples was found to be small with less than 7%, which is below the CV standard of 20%. (B) When it was tested the peak area ratio variability of HER2 (VLQGLPR (SEQ ID NO: 1)) peptide and JAM1 (VTFLPTGITFK (SEQ ID NO: 3)) peptide in the remaining 5 conditions based on 0 hours in low-QC and medium-QC samples, the recovery was found to be 80% - 120 % which is within the standard of the guideline.

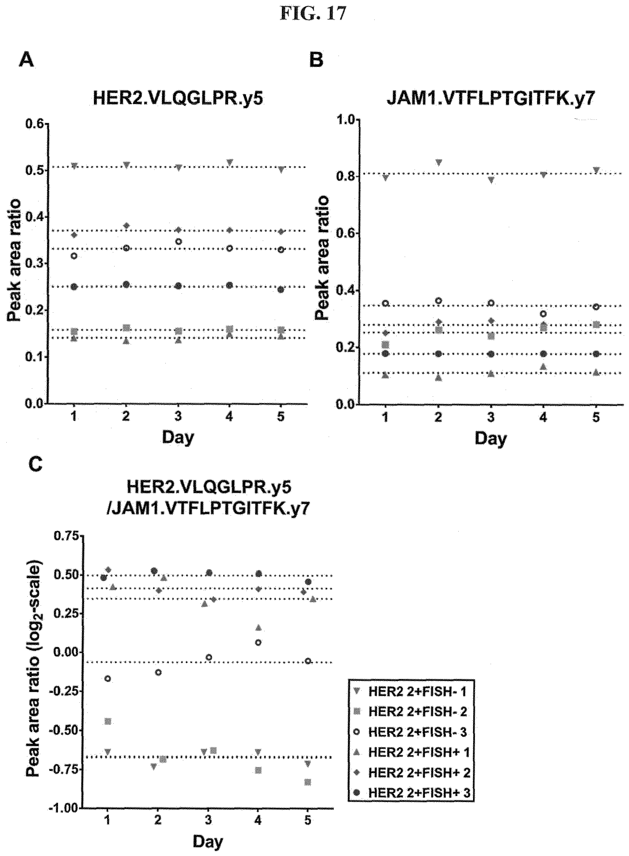

FIG. 17 is a result of testing the reproducibility of the present HER2 (VLQGLPR (SEQ ID NO: 1)) peptide and the JAM1 (VTFLPTGITFK (SEQ ID NO: 3)) peptide in accordance with the Clinical Proteomic Tumor Analysis Consortium (CPTAC) guideline. A total of 6 samples of HER2 2+FISH- (n=3) and HER2 2+FISH+ (n=3) were tested each day from sample preparation to MRM-MS analysis for 5 consecutive days, and then the variability of the peak area ratios of HER2 (VLQGLPR (SEQ ID NO: 1)) and JAM1 (VTFLPTGITFK (SEQ ID NO: 3)) peptides were measured. The variability over the 5 days of testing in the HER2 (VLQGLPR (SEQ ID NO: 1)) peptide (A) and JAM1 (VTFLPTGITFK (SEQ ID NO: 3)) peptide (B) peak area ratios in the 6 samples was found to be small with CV<20%. The values of the HER2 (VLQGLPR (SEQ ID NO: 1)) peptide normalized by the JAM1 (VTFLPTGITFK (SEQ ID NO: 3)) peptide also showed small variability with a CV < 20% in the 5 days of testing. Also, it was found that the normalized value was higher in the HER2 2+FISH+ samples than the three HER2 2+FISH- samples (C).

## DETAILED DESCRIPTION OF THE EMBODIMENTS

In one aspect of the present disclosure, there is provided a method for determining the expression level or the amount of HER2 protein using mass spectrometry in a breast cancer sample, and a method of scoring HER2 status using the same.

In one embodiment, the present method relates to measuring the expression level of HER2 of a breast cancer sample in need thereof *in vitro* for example to provide an information for HER2 status scoring, or for determining the HER2 score of the breast cancer sample based thereon, the method comprising the steps of:

determining an amount of a specific HER2 peptide fragment prepared from the breast cancer sample digested with a protease; and as a normalization factor, determining an amount of the peptide VTFLPTGITFK (SEQ ID NO: 3) from JAM1; and

normalizing the amount of the specific HER2 peptide with the normalization factor to provide a normalized amount of the HER2 peptide;
wherein the specific HER2 peptide fragment is VLQGLPR (SEQ ID NO: 1) or FVVIQNEDLGPASPLDSTFYR (SEQ ID NO: 2); and
wherein the amount of the peptide fragment is determined by a mass spectrometry.

The mass spectrometry which may be used in the method according to the present disclosure may include tandem mass spectrometry, ion trap mass spectrometry, triple quadrupole mass spectrometry, hybrid ion trap/quadruple mass spectrometry and/or time-of-flight mass spectrometry. In this case, the mass spectrometry mode used may be, for example, Selected Reaction Monitoring (SRM) or Multiple Reaction Monitoring (MRM).

In one embodiment, in particular an MRM mode is used. The principle of MRM mass spectrometry is to select a peptide (precursor ion, MS1) having a particular mass to charge (m/z) specific for each target protein after hydrolysis of all selected target proteins into peptides. From the fragments generated when the peptides collide (Quadruple 2, Q2), the fragments (fragmentation ion, MS2) having a particular mass with a specific m/z are selected. Each precursor ion/fragment ion pair obtained from MS1/MS2 is called a transition specific for a target protein (mass fingerprint specific for target protein), and when these transitions are measured for all target proteins (over 300 proteins), the amount of all target proteins present in a sample can be simultaneously quantified relatively or absolutely. For relative or absolute quantification, SIS (having the same amino acid sequence as the corresponding target only with isotopic substitutions) peptide is used as a standard material. In this case, since the amount of the input standard substance (SIS peptide) corresponding to the sample (target) to be measured is known, the amount of target peptide can be proportionally calculated. The transition that passes through MS2 is converted into a digital signal at the detector and converted into a peak chromatogram, and thus by calculating the peak area, the relative and absolute quantitative analysis can be performed.

HER2 is a signal receptor protein expressed on epithelial cells, and in general, HER2 receptor plays a role in the control of the growth, division and repair of healthy cells. However, in some cancers, HER2 protein is overexpressed, which also involves the amplification of HER2 gene. When HER2 is overexpressed in certain cancers, it is important to determine the exact expression status of HER2 because a therapeutic agent targeted to HER2 may be considered. The method currently used for determining the HER2 status is IHC (Immunohistochemistry) and FISH (Fluorescent in situ Hybridization), but the process is complicated and particularly time-consuming and expensive. First, the HER2 score is indicated as 0 (negative), 1+ (negative), 2+ (borderline), or 3+ (positive-Her2 protein overexpression) depending on the IHC test results. FISH is used to test whether the HER2 gene is amplified or not, and indicated as amplification positive or amplification negative. FISH is also performed on the borderline samples classified as 2+ to further classify the borderline sample as 2+FISH+ or 2+FISH-. The samples with FISH positive (2+FISH+), and 3+ respond to anti-HER2 therapy.

The method according to the present disclosure can accurately determine the status of HER2 without performing FISH. Herein, it was found that 2+FISH- and 2+FISH+, which before only can be distinguished by FISH, can be distinguished accurately by the present method using VLQGLPR (SEQ ID NO: 1) or FVVIQNEDLGPASPLDSTFYR (SEQ ID NO: 2) peptide from the HER2 protein and the peptide VTFLPTGITFK (SEQ ID NO: 3) from JAM1 (Uniprot ID Q9Y624)as a standard peptide for normalization with high sensitivity and specificity.

In one embodiment, through MRM-MS analysis using FFPE tissue samples from breast cancer patient and Agilent 6400 series, the expression level of HER2 protein may be determined based on the qualitative analysis of the peaks and the peak area ratio of the two proteins, HER2 and JAM1 as follows: the peak of the 3 product ion (y6, 683.4199+; y5, 570.3358+; y4, 442.2772+) fragmented from the HER2 peptide (VLQGLPR (SEQ ID NO: 1), 391.7478++) in collision energy ragne of 3.1 to 23.1, and the peak of the 3 product ion (y9, 1023.5873+; y8, 876.5189+; y7, 763.4349+) fragmented from JAM1 peptide (VTFLPTGITFK (SEQ ID NO: 3), 612.3554++) of the epithelial cell-specific protein in collision energy range of 10 to 30 to be used as a normalization factor representing the tumor content. Through this, the HER2 status can be determined from 0 to 3+, and the HER2 2+FISH- group and the HER2 2+FISH+ group, which cannot be distinguished by traditional methods, can also be distinguished. Also, based on the HER2 quantitative analysis results, the breast cancer can be classified as either HER2-positive or HER2-negative, which can be used to determine whether to use Herceptin (traszumab) in therapy. In other embodiment, through MRM-MS analysis using FFPE tissue samples from breast cancer patient and Agilent 6400 series, the expression level of HER2 protein may be determined based on the quatitative analysis of the peaks and the peak area ratio of the two proteins, HER2 and JAM1 as follows: the peak of the 3 production (y9, 1085.5262+; y8, 998.4942+; b10, 1115.5732+) fragmented from the HER2 peptide (FVVIQNEDLGPASPLDSTFYR (SEQ ID NO: 2), 790.0655+++) in collision energy ragne of 3.6 to 23.6, and the peak of the 3 product ion (y9, 1023.5873+; y8, 876.5189+; y7, 763.4349+) fragmented from JAM1 peptide (VTFLPTGITFK (SEQ ID NO: 3), 612.3554++) of the epithelial cell-specific protein in collision energy range of 10 to 30 to be used as a normalization factor representing the tumor content.

Through this, the HER2 status can be determined from 0 to 3+, and the HER2 2+FISH- group and the HER2 2+FISH+ group, which cannot be distinguished by traditional methods, can also be distinguished. Also, based on the HER2 quantitative analysis results, the breast cancer can be classified as either HER2-positive or HER2-negative, which can be used to determine whether to use Herceptin (traszumab) in therapy.

In addition, as a normlization factor, the number of tumor cells contained in the predetermined area of a breast cancer sampe was used. For this, in order to correct the differences among the breast cancer samples in the tumor area and tumor cells contained therein, a standard curve is prepared for the amount of extracted protein per tumor cell number, and assign the number of tumor cells in the tumor area into the standard curve to estimate the amount of protein extracted from a predetermined tumor area. Then the number of slides of the breast cancer sample used for analysis is determined in a way to have the similar amount of protein extracted among different samples, enabling more accurate quantitative analysis.

In other aspect, the present disclosure is also directed to a method of determining a HER2 score of a breast cancer sample: 0, 1+, 2+FISH-, 2+ FISH+, or 3+ using the assay results as described above. According to one embodiment, the method comprises correlating the normalized amount of HER2 peptide with any one of the HER2 score of the breast cancer: 0, 1+, 2+FISH-, 2+FISH+, or 3+, wherein the correlation step, to be used as a control, includes the steps of a) determining a normalized amount of HER2 peptide from breast cancer samples representing each of the HER2 scores for which the HER2 score has been determined by IHC(Immunohistochemistry) and FISH (Fluorescent In Situ Hybridization) so as to set a threshold value for each the HER2 scores; and b) comparing the normalized HER2 peptide amount from the breast cancer sample in need thereof with the threshold value determined in a) to assign the score to the sample in need thereof.

In one embodiment, as shown in FIG. 10, the threshold value is determined as the value at which the Youden index (Ruopp et al. Biom J. 2008 Jun; 50(3): 419-430) (J index) becomes maximum (J index = sensitivity + specificity -1) in which the normalized HER2 peptide quantitative analysis value is converted to a binomial log (log2), and then an ROC curve is generated that distinguishes adjacent HER2 scores to obtain specificity and sensitivity.

In one embodiment, when the sequence of SEQ ID NO: 1 is used as a HER2 peptide and a binomial log value of the quantitative analysis values is used, samples having a binomial log value of less than -3.4690 is classified as HER2 0, -3.4690 or more and less than -2.3603 as HER2 1+, -2.3603 or more and less than -1.9241 as HER2 2+FISH-, -1.9241 or more and less than -0.2897 as HER2 2+FISH+, -0.2897 or more as HER2 3+. Further, when the present invention is applied in clinical settings, in addition to the detailed HER2 scoring, it is important to distinguish between a group that requires Herceptin (traszumab) targeted therapy (HER2-positive breast cancer group) and a group that does not (HER2-negative breast cancer group). As shown in FIG. 11, based on -1.9241 which is the binary log value of the normalized quantitative analysis value of HER2 peptide, the samples having a value less than -1.9241 can be classified as HER2-negative breast cancer and the sample having a value 1.9241 or more can be classified as HER2-positive breast cancer.

In other embodiment, when the sequence of SEQ ID NO: 2 is used as a HER2 peptide and a binomial log value of the quantitative analysis values is used, samples having a binomial log value of of less than -1.8208 is classified as HER2 0, -1.8208 or more and less than -1.2673 as HER2 1+, -1.2673 or more and less than -0.5299 as HER2 2+FISH-, -0.5299 or more and less than 0.9543 as HER2 2+FISH+, and 0.9543 or more as HER2 3+.

Accordingly, those skilled in the art would be able to perform MRM-MS analysis-based quantitative analysis of the peptides such as HER2 peptides and JAM1 and other normalization peptides disclosed in the present invention, and then determine a threshold value such as shown in FIG. 11 and determine whether the target based Herceptin (traszumab) therapy is needed. The threshold value may be different depending on the type of HER2 peptides and normalizatoin peptides employed. As mentioned above, the threshold value is determined as the value at which the Youden index (Ruopp et al. Biom J. 2008 Jun; 50(3): 419-430) (J index) becomes maximum (J index = sensitivity + specificity -1) in which the normalized HER2 peptide quantitative analysis value is converted to a binomial log (log2), and then an ROC curve is generated that distinguishes adjacent HER2 scores to obtain specificity and sensitivity.

In one embodiment, the sample used in the method according to the present disclosure is FFPE (Formalin-Fixed, Paraffin-Embedded). FFPE tissue samples are the most frequently used clinical samples to diagnose breast cancer and determine whether to apply targeted chemotherapy, and samples in various stages can be stored and easily available at low cost. Therefore, through quatification of HER2 peptided and epithelial cell-specific peptided as a normalization factor by MRM-MS analysis using FFPE tissue samples from breast cancer patients, HER2 scoring can be measured more accurately and reproducibly than conventional methods.

In one embodiment, the protein for use in mass spectrometry in the method according to the present invention is digested with a trypsin. In one embodiment, when the FFPE tissue sample used in the method according to the present disclosure, the sample is treated with trypisin particulary for about 4 hours.

The present disclosure is further explained in more detail with reference to the following examples. These examples, however, should not be interpreted as limiting the scope of the present invention in any manner.

## EXMAPLES

## MATERIALS AND METHODS

### 1. Clinical Tissue Samples and Characteristics thereof

The FFPE tissue samples used in the present disclosure were collected from a total of 210 breast cancer patients who underwent mastectomy, and the written informed consent was obtained from all the patients. The 210 samples were classified based on their HER2 status as follows: HER2 0 (30 cases), HER2 1+ (30 cases), HER2 2+FISH- (61 cases), HER2 2+FISH+ (59 cases), HER2 3+ (30 cases), and the clinical pathological information for each sample is shown in Table 2.

[Table 2]

| | HER2 status | | | | |
|---|---|---|---|---|---|
| Group | HER2 0 (n=30) | HER2 1+ (n=30) | HER2 2+/ FISH-negative (n=61) | HER2 2+/ FISH-positive (n=59) | HER2 3+ (n=30) |
| Age (years) | 53.80 ± 9.57 | 53.60 ± 11.39 | 48.62 ± 9.86 | 54.22 ± 10.95 | 50.57 ± 11.66 |
| FISH status | | | | | |
| Negative | 0 | 2 | 61 | 0 | 0 |

(continued)

| Group | | HER2 status | | | | |
|---|---|---|---|---|---|---|
| | | HER2 0 (*n*=30) | HER2 1+ (*n*=30) | HER2 2+/ FISH-negative (*n*=61) | HER2 2+/ FISH-positive (*n*=59) | HER2 3+ (*n*=30) |
| | Positive | 0 | 0 | 0 | 59 | 20 |
| | NA | 30 | 28 | 0 | 0 | 10 |
| Estrogen Receptor | | | | | | |
| | Negative | 18 | 7 | 4 | 20 | 20 |
| | Positive | 12 | 23 | 57 | 39 | 10 |
| Progesterone Receptor | | | | | | |
| | Negative | 21 | 7 | 11 | 30 | 24 |
| | Positive | 9 | 23 | 50 | 29 | 6 |
| Subtype | | | | | | |
| | HER2 | 0 | 0 | 0 | 21 | 20 |
| | Luminal A | 10 | 23 | 53 | 0 | 0 |
| | Luminal B | 3 | 0 | 4 | 38 | 10 |
| | TNBC | 17 | 7 | 4 | 0 | 0 |
| Nuclear grade | | | | | | |
| | 1 | 0 | 1 | 0 | 1 | 0 |
| | 2 | 5 | 15 | 38 | 13 | 5 |
| | 3 | 25 | 14 | 22 | 45 | 25 |
| | NA | 0 | 0 | 1 | 0 | 0 |
| Histological grade | | | | | | |
| | I | 1 | 3 | 6 | 1 | 0 |
| | II | 4 | 16 | 37 | 25 | 8 |
| | III | 25 | 11 | 17 | 33 | 22 |
| | NA | 0 | 0 | 1 | 0 | 0 |
| Tumor size | | | | | | |
| | < 2.0 cm | 7 | 12 | 28 | 30 | 19 |
| | 2.0 - 4.9 cm | 21 | 17 | 32 | 27 | 10 |
| | ≥ 5.0 cm | 2 | 1 | 1 | 2 | 1 |

FISH, fluorescence in situ hybridization

## 2. Treatment of breast cancer tissue samples and preparation of FFPE block.

The breast cancer lesion tissues collected from the mastectomy were fixed by placing them in a formalin solution to prevent tissue damage and store for long periods. Before use, the formalin solution on the surface of the tissues were removed by washing the fixed tissues under running water. Subsequently, the tissues were dehydrated by immersing them in water and ethanol in the following order: water → 50% ethanol → 70% ethanol → 85% ethanol → 100% ethanol. Next, the tissues were treated with Xylene to remove the dehydrating agent used to facilitate paraffin penetration. Then the tissues were embedeed in paraffin wax. Through this process, the paraffin wax was inflitrated into the tissue and hardened to protect the tissue and the tisuse can be stored for long periods.

### (1) Preparation of Breat Cancer FFPE tissue section and H&E staining

FFPE tissue sections were obtained by sectioning FFPE tissue blocks to a thickness of 10 μm using a microtome, and 6 sections were prepared for each sample. One of 6 FFPE tissue sections was used for nuclear and cytoplasmic staining of the cells by H&E (Hematoxylin & Eosin) agent. Then the pathologist examined the H&E-stained FFPE tissue sections and marked the tumor formation site.

**(2) Deparaffinization, rehydration and protein denaturation of the breat cancer FFPE**

One mg of Rapigest was dissolved in 210μL of 30mM Tris-Cl (pH 8.5) solution to prepare 0.5% Rapigest solution. The paraffin was removed from the breast cancer FFPE tissue section and rehydrated by immersing the section for 3 mins in the respective solution as follows: Xylene → Xylene → 100% ethanol → 100% ethanol → 85% ethanol → 70% ethanol → 50% ethanol → water. Then the FFPE tissue sections were treated with 200 μL of 30 mM Tris-Cl (pH 8.5) solution to prevent the tissue from drying out. Then the breast cancer tissue was scraped from the breast cancer FFPE tissue section using a scraper and put into 100 μL of 0.5% Rapigest in an Eppendorf® tube followed by sonication for 10 seconds. Then, it was heated (1,000 rpm, 95 °C) for 30 minutes in a ThermoMixer®. After cool down to room temperature, it was spin-down to move all the solutions to the bottom of the tube. Then the tissues were disrupted using a probe sonicator (Amplitude: 30%, pulses ON and OFF, repeated 20 times for 4 seconds each) and centrifuged at 15,000 rpm for 30 minutes (maintained at 20 °C). After centrifugation, 80 μL of the supernatant was transferred to a new Eppendorf® tube, and the remaining supernatant was transferred to separate Eppendorf® tube for BCA analysis.

**(3) RapiGest™ in-solution digeston of breast cancer FFPE tissue samples**

Twenty microliter of 200mM DTT were added to the tube containing 80 μL of the supernatant prepared as above, which was then vortexed to mix the denatured protein with DTT and was spined down. Then, the tube was heated (1,000 rpm, 60° C.) for 1 hour in a ThermoMixer®. Then 20μL of 240mM IAA were added thereto and mixed by vortexing followed by spin-down. Then it was left at room temperature for 1 hour by blocking the light by wrapping it with aluminum foil. Then 60 μL of 0.067 μg/μL trypsin (trypsin: protein = 1:50) were added thereto, vortexted and spinned down, after which it was heat treated in ThermoMixer® for 4 hours (1,000 rpm, 37° C).

**(4) Remove of RapiGest™ and Preparation of sample for MRM-MS analysis**

Twenty microliter of 30% formic acid were added to the trypsin digested sample as above and vortexed gently, and the acidity was measured using CR paper. Then the sample was incubated in a ThermoMixer® (1,000rpm, 37°C) for 30 minutes, and centrifuged for 1 hour (15,000rpm, 4 °C) to obtain 200 μL of supernatant. Then 100 μL of the supernatent was transferred to a new Eppendorf® tube for MRM-MS analysis, and the remaining 100 μL was transferred to a separate tube for peptide yield measurment by tryptophan fluorescence analysis.
For MRM-MS analysis, 90 μL out of 100 μL of the supernatant was transferred into a glass vial, and 10 μL of 150 fmol/ μL SIS peptide mixture was added thereto to make a total of 100 μL mixture. The mixture was then vortexed for about 1 minute, abd spinned down.

**3. Capillary liquid chromatography for peptide separation**

Prior to MRM-MS analysis, 6 HER2 peptides, 19 epithelial cell-specific peptides and 30 housekeeping peptides were separated by liquid chromatography (1260 Capillary-flow liquid chromatography system; Agilent Technologies, Santa Clara, CA, USA).
For this, impurities (residual RapiGest™ and salt) contained in the sample were removed using a guard column (2.1 × 15.0 mm, 1.8 μm, 80 Å), and then an analytical column (0.5 × 35.0 mm, 3.5 μm, 80 Å) was used to perform peptide separation. Solvent A (0.1% formic acid in water) and solvent B (0.1% formic acid in acetonitrile) were used on an LC (liquid chromatography) system consisting of two columns. While flowing 3% solvent B at a flow rate of 40 μL/min for 10 minutes, 40 μL of the sample was injected into the guard column during which the impurities contained in the sample were removed. After switching the valve, the peptides attached to the guard column moved to the analytical column and attached thereto. By flowing solvent B from 3% to 50% gradient at a flow rate of 40 μL/min, the peptides attached to the analytical column were eluted. After washing the column by flowing 60% solvent B at a flow rate of 40 μL/min for 2 minutes, solvent B was flowed from 60% to 3% gradient at a flow rate of 40 μL/min for 1 minute to readjust the column to equilibrium. After equilibration for 4 minutes, the valve was changed to the original position and reconditioning of the guard column and the analytical column occurred simultaneously for 10 minutes. Total sample analysis time was 70 minutes. Then, after injecting the sample into the LC system, the autosampler injector needle and tube were washed with 50% acetonitrile aqueous solution.

**4. Online desalting and MRM-MS analysis**

MRM-MS quantitative analysis was performed using an Agilent 6400 series. Here, the ionized peptides flowed into Quadrupole 1 (Q1), and a precursor ions with a specific m/z (mass to charge ratio) were selected in Q1. The precursor ions that passed through the Q1 filter were decomposed into product ions due to the fragmentation of the ions by electrical energy in the quadrupole 2 (Q2). The product ions flowed into Quadrupole 3 (Q3), where product ions with a specific m/z were selected, moved to a detector, and converted into a digital signal to appear as a peak chromatogram. Relative and absolute quantitative analysis were then performed by calculating the peak area (FIG. 1).

### 5. Post-processing of raw files and statistical analysis

The raw file was introduced into the skyline software for post-processing of the raw file derived from LC-MS/MS analysis. In order to calculate quantitative analysis values for 6 HER2 peptides, 19 epithelial cell-specific peptides, and 30 housekeeping peptides, the peak integration for the corresponding peptide peaks was performed (designating the peak corresponding to the retention time of each peptide). The peak area ratio of the SIS peptide (Heavy peptide) peak and the endogenous peptide (Light peptide) peak within the retention time was calculated. For statistical analysis, Mann-Whitney test, area under the receiver operating curve, correlation analysis, etc. were performed using IBM SPSS and MedCalc software and visualized using Prism software.

### 6. Selection of normalization factors that can noramlize quantitative analysis values of HER2 protein

To normalize quantitative data of HER2 peptide, Five normalization factors (tumor tissue area, number of cells in tumor tissue area, total protein amount, total peptide amount, 19 epithelial cell-specific peptides and 30 housekeeping peptides) that can represent tumor contnes were selected. Two of these normalization factors (tumor tissue area and the number of cells in the tumor tissue area) were measured by embedding H&E staining tissue section images in Aperio ImageScope, ver. 12.3.0.5056 (Leica Biosystems, Buffalo Grove, IL, USA). In Aperio software, after the tumor area was annotated using a pen tool, the area and total cell count of the annotated region were measured with the nuclear counting algorithm.

The third normalization factor was the total amount of protein extracted from the tumor region of the 10 $\mu$m-thick FFPE tissue section, and the total amount of protein was deternubed by BCA analysis.

The fourth normalization factor was the amount of peptide, which is the final product of trypsinization, and the total amount of peptides was determined by tryptophan fluorescence analysis.

As a final normalization factor, the peak area ratio (area of light peptide/area of heavy peptide) of 19 epithelial cell-specific peptides and 30 housekeeping peptides quantified by MRM-MS analysis was determined.

The normalized HER2 peptide quantitative value is the value obtained by dividing the peak area ratio of the HER2 peptide by the noramlization factor, and this value was compared with the IHC and FISH results.

The accuracy and AUC values when the (HER2 FISH-) group and the (HER2 FISH+) group can be discriminated were determined using the HER2 peptide quantitative analysis values normalized by five standardization factors. Among them, the normalizaton factor with the highest Accuracy and AUC values was selected as the optimal standardization factor. Then It was used to test that whether the overall HER2 status can be significantly ($p<0.05$) distinguished using the HER2 peptide level normalized with the optimal standardization factor.

### EXAMPLE 1: Flowchart for HER2 Status Classification in MRM-MS Analysis of Clinical Samples

For HER2 scoring through MRM-MS analysis, FFPE tissue sections from a total of 210 breast cancer patients were used. HER2 status consists of a total of 5 types (0, 1+, 2+FISH-, 2+FISH+, 3+), and the number of individual samples according to HER2 status was varied (see Table 3). One hundred twenty samples classified as HER2 2+ (HER2 equivocal) by IHC were finally determined to be HER2 negative or positive through FISH analysis.

[Table 3]

| Breast Cancer FFPE tissue (210) | | | | |
|---|---|---|---|---|
| HER2 (0) | HER2 (1+) | HER2 (2+FISH-) | HER2 (2+FISH+) | HER2 (3+) |
| 30 | 30 | 61 | 59 | 30 |

Then, the tissue samples collected from the breast cancer patients were made into FFPE blocks, sectioned into consecutive sections with a thickness of 10 $\mu$m. One of the FFPE tissue sections was subjected to H&E staining and the image was used for counting the number of cells present in the tumor region using the nuclear counting algorithm of Aperio software. Next, the expected amount of extracted protein was determined by assign the number of cells above to the equation for the standard curve for the amount of protein extracted per numbers of the cells. Based on the calculated amount of protein, the number of FFPE tissue slides to obtain 150 µg of protein was determined. The FFPE tissue sections were deparaffinized and rehydrated, and the proteins were extracted and digested with trypsin to obtain peptides. Then, HER2 peptides and normalization peptides were measured by MRM-MS analysis to finally distinguish the HER2 status (refer to the flowchart of FIG. 2).

**EXAMPLE 2: Screening of HER2 peptides, epithelial cell-specific peptides and housekeeping peptides that can be detectable from the breast cancer FFPE tissue samples**

Through a literature search to select epithelial cell-specific proteins and housekeeping proteins detectable from breast cancer FFPE tissue samples, 46 proteins were selected as a possible normalization proteins from breast cancer FFPE tissue samples.

Here it was confirmed through matching analysis with the MS/MS library obtained with the National Institute of Standards and Technology (NIST) that 123 peptides corresponding to a total of 47 proteins including HER2 protein and 46 normalization proteins were detectable by mass spectrometry.

Next, in order to select the peptides that are reproducibly detected in MRM-MS analysis, semiquantitative MRM-MS analysis was performed using a pooled sample of 10 breast cancer FFPE tissues corresponding to HER2 3+. As a results, 37 proteins (62 peptides) were selected as detectable peptides.

Based on this, 62 SIS (unpurified stable-isotope-labeled standard) peptides were synthesized, and a mixture of the SIS peptides was prepared.

Then, the SIS peptide mixture was spiked into the pooled breast cancer FFPE tissue samples, and MRM-MS was repeated three times to perform the analysis, and using the result, AuDIT (Automated detection of inaccurate and imprecise transitions analysis) was performed to remove erroneously detected ions, and a maximum of three transitions detected with reproducibly high intensity in each peptide were selected.

As a result, finally 31 proteins (55 peptides) were selected as final detectable targets through MRM-MS analysis in breast cancer FFPE tissue samples. Among them, 30 normalization proteins (49 peptides) were included (Table 4).

[Table 4]

| Uniprot ID | Uniprot name | Peptide sequence | SEQ ID NO | Uniprot ID | Uniprot name | Peptide sequence | SEQ ID NO |
|---|---|---|---|---|---|---|---|
| P27797 | CALR | FVLSSGK | 29 | Q15084 | PDIA6 | TGEAIVDAALSALR | 13 |
| | | FYALSASFEPFSNK | 30 | | | ELSFGR | 14 |
| P23528 | COF1 | NIILEEGK | 22 | P23284 | PPIB | IGDEDVGR | 45 |
| O60716 | CTND1 | GYELLFQPEWR | 10 | P51149 | RAB7A | VIILGDSGVGK | 11 |
| O00571 | DDX3X | SFLLDLLNATGK | 31 | | | EAINVEQAFQTIAR | 12 |
| P15924 | DESP | AELIVQPELK | 27 | P62826 | RAN | FNVWDTAGQEK | 17 |
| Q9Y624 | JAM1 | VTFLPTGITFK | 3 | P35268 | RL22 | AGNLGGGVVTIER | 23 |
| P04406 | G3P | GALQNIIPASTGAAK | 25 | | | ITVTSEVPFSK | 46 |
| | | VPTANVSVVDLTCR | 32 | P62888 | RL30 | SLESINSR | 15 |
| P06396 | GELS | HVVPNEVVVQR | is | | | LVILANNCPALR | 16 |
| | | TGAQELLR | 19 | P62910 | RL32 | AAQLAIR | 28 |
| P05783 | K1C18 | STFSTNYR | 33 | P32969 | RL9 | TILSNQTVDIPENVDITLK | 47 |
| | | VIDDTNITR | 34 | | | GVTLGFR | 48 |
| | | ASLENSLR | 35 | P46783 | RS10 | IAIYELLFK | 49 |
| P08727 | K1C19 | ILGATIENSR | 36 | P62277 | RS13 | GLTPSQIGVILR | 50 |
| | | AALEDTLAETEAR | 37 | | | LILIESR | 2.4 |
| P08729 | K2C7 | SAYGGPVGAGIR | 38 | P62249 | RS16 | GGGHVAQIYAIR | 51 |
| | | EVTINQSLLAPLR | 39 | P08708 | RS17 | VCEEIAIIPSK | 21 |
| | | LPDIFEAQIAGLR | 40 | P07437 | TBB5 | ISVYYNEATGGK | 5 |
| P05787 | K2C8 | ISSSSFSR | 41 | | | ALTVPELTQQVFDAK | 4 |
| | | ASLEAAIADAEQR | 42 | P55072 | TERA | WALSQSNPSALR | 20 |
| P00338 | LDHA | VTLTSEEEAR | 26 | P18206 | VINC | AIPDLTAPVAAVQAAVSNLVR | 8 |
| Q96NY8 | NECT4 | YEEELTLTR | 43 | | | AQQVSQGLDVLTAK | 9 |
| P13667 | PDIA4 | FDVSGYPTLK | 44 | | | SLGEISALTSK | 6 |
| | | | | | | ELTPQVVSAAR | 7 |

**EXMAPLE 3: Development (of MRM Analysis)**

A reverse calibration curves was generated using the pooled 18 HER2 3+ FFPE tissue samples to determine the detection and quantitation limits (LOD, LOQ) and other parameters (Linearity, LLOQ, ULOQ) of the peptides for MRM-MS analysis (FIG. 3). Each point on the calibration curve was generated by performing 3 independent MRM-MS analysis. Linearity, LOD (Limit of detection), LOQ (Limit of quantification), LLOQ (Lower limit of quantification) and ULOQ (Upper limit of quantification were derived from the reverse calibration curve for the y5 product ion of the VLQGLPR (SEQ ID NO: 1) peptide of the HER2 protein and the y7 product ion of the VTFLPTGITFK (SEQ ID NO: 3) peptide of the JAM1 protein as follows (FIG. 3).

The SIS peptide concentration points of the reverse calibration curve for the VLQGLPR (SEQ ID NO: 1) peptide of the HER2 protein and the VTFLPTGITFK (SEQ ID NO: 3) peptide of the JAM1 protein were adjusted based on the purity of the spiked SIS peptide (FIG. 13), and the values for the y5 product ion of the VLQGLPR (SEQ ID NO: 1) peptide of the HER2 protein was as follows: LOD = 0.399 fmol, LOQ = 0.985 fmol, LLOQ = 0.647 fmol, ULOQ = 1325 fmol, linear regression value ($R^2$) = 0.999 of the equation Y=1.029X - 4.732 for the inverse calibration curve.

The values for y7 product ion of the VTFLPTGITFK (SEQ ID NO: 3) peptide of the JAM1 protein was as follows: LOD = 2.212 fmol, LOQ = 5.043 fmol, LLOQ = 2.587 fmol, ULOQ = 1325 fmol, linear regression value ($R^2$) = 0.998 of the equation Y=1.113X- 3.772 for the inverse calibration curve.

The CV range of the 3 independent analysis on the inverse calibration curve of the y5 product ion of the VLQGLPR (SEQ ID NO: 1) peptide of the HER2 protein was 4.02% to 19.21%, and the CV range of the 3 independent analysis on the inverse calibration curve of the y7 product ion of the VTFLPTGITFK (SEQ ID NO: 3) peptide of the JAM1 protein was 4.91 % to 23.45% (Table 5).

[Table 5]

| Protein Peptide Transition | Measurements | Blank sample | Calibrator 1 | Calibrator 2 | Calibrator 3 | Calibrator 4 | Calibrator 5 | Calibrator 6 | Calibrator 7 | Calibrator 8 | Calibrator 9 | Calibrator 10 | Calibrator 11 | Calibrator 12 |
|---|---|---|---|---|---|---|---|---|---|---|---|---|---|---|
| HER2 VLQGLPR (SEQ ID NO: 1) y5 | Concentration point (fmol) | N/A | 0.65 | 1.29 | 2.59 | 5.18 | 10.35 | 20.70 | 41.41 | 82.82 | 165.64 | 331.28 | 662.55 | 1325.10 |
| | Mean Area ratio (Heavy/Light) | 0.00 | 0.03 | 0.05 | 0.10 | 0.20 | 0.40 | 0.88 | 1.54 | 3.65 | 7.03 | 16.99 | 28.36 | 64.45 |
| | SD | 0.00 | 0.00 | 0.01 | 0.01 | 0.03 | 0.02 | 0.09 | 0.08 | 0.44 | 1.35 | 0.76 | 1.71 | 5.03 |
| | CV (%) | 64.27 | 5.45 | 18.30 | 9.62 | 16.92 | 4.02 | 10.22 | 5.22 | 11.95 | 19.21 | 4.46 | 6.02 | 7.80 |
| JAM1 VTFLPTGITFK (SEQ ID NO: 3) y7 | Concentration point (fmol) | N/A | 2.59 | 5.17 | 10.35 | 20.70 | 41.39 | 82.78 | 165.56 | 331.13 | 662.25 | 1324.50 | N/A | N/A |
| | Mean Area ratio (Heavy/Light) | 0.06 | 0.23 | 0.49 | 0.80 | 1.94 | 5.09 | 11.46 | 21.06 | 44.51 | 113.44 | 204.75 | N/A | N/A |
| | SD | 0.04 | 0.02 | 0.11 | 0.18 | 0.34 | 0.60 | 1.48 | 4.03 | 6.60 | 5.57 | 33.24 | N/A | N/A |
| | CV (%) | 57.75 | 8.02 | 23.45 | 21.92 | 17.55 | 11.88 | 12.95 | 19.13 | 14.83 | 4.91 | 16.24 | N/A | N/A |

SD, standard deviation; CV, coefficient of variation; N/A, Not available.

**EXAMPLE 4: Determination of optimal incubation time with trypsin**

Since the MRM-MS analysis result of the HER2 peptides may vary depending on the reaction time with trypsin, the trypsin reaction time test was performed as follows to select an optimal reaction time with trypsin that can produce optimal strength of the HER2 peptide detected in MRM-MS analysis.

The trypsin reaction time for the experiment was set to 8 points (1, 2, 4, 8, 12, 16, 20, 24 h), and because at least 100 μg of protein is required for each point, one HER2 3+ FFPE tissue sample with a relatively large tumor tissue area (from which about 200 μg of protein may be extracted) from each of 6 cases was treated with 0.5% RapiGest™ in solution digestion. After digestion, 10 μL of each sample was pooled, and the concentration was measured through BCA analysis. After sequential reaction with DTT and IAA using 80 μL of the supernatant of each of the 6 samples, 60 μL of 0.067 μg/μL trypsin was added (trypsin: protein = 1:50) to each tube, vortexed and spin-down was performed. Six samples were then pooled into one tube and then aliquoted in the same volume to eight separate tubes for digestion with trypsin. The trypsin reaction time (1, 2, 4, 8. 12, 16, 20, 24 hours) was set differently for each of the 8 tubes, and the trypsin reaction was stopped at the end of each time set, and then formic acid was added to a final concentration of 3% to remove RapiGest™. Eight samples were mixed with SIS peptide (90 μL of sample + 10 μL of SIS peptide), and 10 μL of each was pooled to prepare a sample. This is to determine the retention time through analysis of the pooled sample, and to analyze the sample according to the trypsin reaction time by scheduled MRM-MS.

The pooled samples were analyzed once by unscheduled MRM-MS. After inserting the raw file into Skyline, peak integration was performed to determine the retention time for each peptide. After preparing the method file for scheduled MRM-MS analysis, 8 individual samples according to the trypsin reaction time were subjected to scheduled MRM-MS analysis (analyzed twice). Among the product ions of the six HER2 peptides, one or two product ions with good detection for each peptide were selected and the peak area ratio (L/H) value was calculated. After comparing the results of each trypsin reaction time, the final optimal reaction time with trypsin was determined to be 4 hours (FIG. 4).

**EXAMPLE 5: Generation of a standard curve for the amount of extracted protein per number of cells**

Because the size of the tumor site varies from sample to sample, the number of FFPE tissue slides that need to be used to extract the same amount of protein from different samples also varies from sample to sample. In addition, the greater the difference in the amount of protein extracted from each sample, the greater the difference in peptide yield between the samples. Therefore, in order to ensure that the same/similar amount of proteins are extracted from each samples, it is necessary to extract proteins from several consecutive tissue sections for the samples with a relatively small tumor area.

In this context, in order to extract a similar/same amount of protein from each sample, it is necessary to objectively determine how many slides to use for each sample for the assay. This was solved by using a standard curve indicating the correlation between the number of cells present in the tumor region and the protein extracted from the tumor region.

Based on the hypothesis that the amount of extracted protein is proportional to the number of cells, proteins was extracted from each of 10 individual samples with different cell numbers in the tumor area in which 3 replicates of FFPE tissue slides were used for each sample. The number of cells in the tumor area of individual FFPE tissue samples was determined by applying H&E staining tissue section images in Aperio software, in which the tumor area was marked using the pen tool, and then the Nuclear counting algorithm was used to determine the number of cells in the tumor tissue area.

Then, the amount of protein in a total of 30 samples was measured by BCA analysis. As a result, it was confirmed that the amount of extracted protein per cell number was linear ($R^2$ = 0.9916), and the CV between the three replicates at 10 points was all less than 20% (Table 6, FIG. 5). The 10 individual samples used to generate a standard curve for the amount of protein extracted per number of cells was found to be representative of the total 210 samples used in this study. This was confirmed by that there were no statistically significant differences beteween the 10 samples and 210 samples in the six histopathological characteristics-tumor area (μm), cell number, intratumoral fat content (%), necrosis area (%), tumor content (%), and tumor-infiltrating lymphocyte (%) (FIG. 14).

[Table 6]

| No. | Cell counts | Extracted proteins (μg) | | |
|-----|-------------|-------------------------|------|--------|
| | | Average | SD | CV (%) |
| 1 | 48968 | 0.00 | 1.30 | 14.49 |
| 2 | 69469 | 12.49 | 0.41 | 3.25 |
| 3 | 120075 | 25.34 | 2.92 | 11.54 |
| 4 | 149723 | 31.59 | 0.23 | 0.72 |

(continued)

| No. | Cell counts | Extracted proteins (μg) | | |
|---|---|---|---|---|
| | | Average | SD | CV (%) |
| 5 | 185695 | 34.12 | 3.07 | 9.00 |
| 6 | 228840 | 66.83 | 6.44 | 9.64 |
| 7 | 379759 | 92.50 | 7.46 | 8.07 |
| 8 | 552041 | 148.54 | 6.80 | 4.58 |
| 9 | 736601 | 165.51 | 3.89 | 2.35 |
| 10 | 1915542 | 406.13 | 25.28 | 6.23 |

SD, Standard deviation; CV, Coefficient of variation

## EXAMPLE 6: Results of MRM-MS analysis of HER2 peptides, epithelial cell-specific peptides, and housekeeping peptides in 210 individual samples

Six HER2 peptides, 19 epithelial cell-specific peptides and 30 housekeeping peptides selected in the previous Example were quantitatively analyzed by MRM-MS analysis of 210 individual samples using Agilent 6400 series.

As a result of determining the correlation between the six HER2 peptides through Spearman rank correlation, it was confirmed that there was a positive correlation between the six HER2 peptides (FIGs. 6 and 7A and 7B). The peptide LLDIDETEYHADGGK (SEQ ID NO: 53) and the peptide ELVSEFSR (SEQ ID NO: 54) showed the lowest Spearman correlation coefficient ($\rho$) of 0.596. The peptides VLQGLPR (SEQ ID NO: 1), GLQSLPTHDPSPLQR (SEQ ID NO: 52) and FVVIQNEDLGPASPLDSTFYR (SEQ ID NO: 2) showed a high Spearman correlation coefficient ($\rho$) of 0.8 or more. All six peptides are from the same HER2 protein, and thus theoretically, the results of MRM-MS quantitative analysis from the peptides should be similar to each other. In this context, the correlation between the quantitative analysis results of six HER2 peptides of the present invention was expressed as a Spearman correlation coefficient, and since all correlations had positive values, it can be seen that the quantitative analysis results from the peptides are similar to each other. The above results showed the correlation results between HER2 peptides quantified in 210 samples analyzed here. Theoretically, the correlation coefficient between the six peptides should be close to 1.000, but since the characteristics of each peptide are different, the correlation between the quantified values by MRM-MS analysis may not be the theoretically expected 1.000. Referring to FIG. 6, in the case of the ELVSEFSR (SEQ ID NO: 54) peptide, the correlation with the other five peptides was found to be relatively low. This means that the yield of the ELVSEFSR (SEQ ID NO: 54) peptide was different from that of the other 5 peptides during the sample preparation process (for example, the ELVSEFSR (SEQ ID NO: 54) peptide produced a yield lower than the other 5 peptides), or the ELVSEFSR (SEQ ID NO: 54) peptide has a different degree of ionization than that of other peptides in MRM-MS analysis. For example, this measns that the ELVSEFSR (SEQ ID NO: 54) peptide has a lower peptide yield during sample pretreatment than the other five peptides, and the ionization is not good during MRM-MS analysis thus lowering the detection efficiency during analysis. For this reason, it was determiend that the ELVSEFSR (SEQ ID NO: 54) peptide is not suitable for HER2 scoring in HER2 quantitative analysis. On the other hand, the VLQGLPR (SEQ ID NO: 1) peptide showed a high correlation coefficient ($\rho$) of 0.8 or more with GLQSLPTHDPSPLQR (SEQ ID NO: 52) and FVVIQNEDLGPASPLDSTFYR (SEQ ID NO: 2), and also showed a relatively high Spearman correlation coefficient ($\rho$) with the other three peptides and thus seleced as HER2 analysis peptide.

## EXAMPLE 7: Agreement between MRM-MS data and IHC/FISH data

Including four normalization factors that can represent tumor conten and peak area ratio (L/H) of the peptides from MRM-MS analysis (peak area ratio of epithelial cell-specific peptides and housekeeping peptides (L/H)), the quantitative values of the six HER2 peptides were divided by a total of five normalization factors to normalize the quantitative values of the six HER2 peptides. Then, using the normalized quantitative values of the six HER2 peptides, a statistical analysis was performed to determine Accuracy and AUC value of the present invention in which the statistical analysis was performed as to distinguish between the HER2 2+FISH- group and the HER2 2+FISH+ group, which cannot be distinguished by the conventional method (HER2 status scoring based on IHC).

Each of the MRM-MS quantitative analysis values of 6 HER2 peptides (MRM analysis values of the peptides) was divided by 53 normalization factors (number of cells in tumor area, tumor tissue area, protein amount, peptide amount, 49 epithelial cell-specific peptides and housekeeping peptide), and the value was used to determine the Accuracy and AUC values for a total of 318 combinations. Among these 318 combinations, the top 30 combinations with an AUC value of 0.800 or higher are shown in Table 7.

AUC values for the 318 combinations ranged from 0.617 to 0.908. When the quantitative analysis result of HER2 peptide was normalized by various normalization factors, it was found that the ability to distinguish between the HER2 2+FISH- group and the HER2 2+FISH+ group was found to be excellent in the order of epithelial cell-specific peptide and housekeeping peptide, the number of cells in the tumor area, peptide amount, protein amount, and tumor tissue area (FIG.8). Among them, it was found that the normalized value, in which the peak area ratio of the three product ions (y6, 683.4199+; y5, 570.3358+; y4, 442.2772+) produced from the fragmentaion of the HER2 peptide (VLQGLPR (SEQ ID NO: 1), intrinsic mass value 391.7478++) in a colliiosn enery range of 3.1 - 23.1 was normalized by the peak area ratio of the three product ions (y9, 1023.5873+; y8, 876.5189+; y7, 763.4349+) produced from the fragmentaion of the epithelial cell-specific protein JAM1 peptide (VTFLPTGITFK (SEQ ID NO: 3), intrinsic mass value 612.3554++) in a colliiosn enery range of 10-30, was the most accurate in distingushing the HER2 2+FISH- group and the HER2 2+FISH+ group as an Accuracy of 88.33% and AUC of 0.908 (FIG. 9). Among 61 HER2 2+FISH- samples, 54 (88.52%) were correctly classified as HER2 negative, and among 59 HER2 2+FISH+ samples, 52 (88.14%) were correctly classified as HER2 positive. Overall, among 120 equivocal HER2 samples, 113 (88.33%) were correctly classified (Table 8).

[Table 7]

| Number | HER2 Peptide sequence | Normalization factor | AUROC value | 95% CI |
|---|---|---|---|---|
| 1 | VLQGLPR (SEQ ID NO: 1) | * PAR of JAM1_VTFLPTGITFK (SEQ ID NO: 3) | 0.908 | 0.842 - 0.953 |
| 2 | VLQGLPR (SEQ ID NO: 1) | PAR of TBB5_ALTVPELTQQVFDAK (SEQ ID NO: 4) | 0.867 | 0.79 3 - 0.922 |
| 3 | VLQGLPR (SEQ ID NO: 1) | PAR of VINC_SLGEISALTSK (SEQ ID NO: 6) | 0.854 | 0.778 - 0.912 |
| 4 | VLQGLPR (SEQ ID NO: 1) | PAR of VINC_ELTPQWSAAR (SEQ ID NO: 7) | 0.853 | 0.777 - 0.911 |
| 5 | VLQGLPR (SEQ ID NO: 1) | PAR of CTND1_GYELLFOPEWR (SEQ ID NO: 10) | 0.852 | 0.775 - 0.910 |
| 6 | VLQGLPR (SEQ ID NO: 1) | PAR of VINC_AIPDLTAPVAAVQAAVSNLVR (SEQ ID NO: 8) | 0.848 | 0.771 - 0.907 |
| 7 | VLQGLPR (SEQ ID NO: 1) | PAR of TBB5_ISVYYNEATGGK (SEQ ID NO: 5) | 0.844 | 0.767 - 0.904 |
| 8 | VLQGLPR (SEQ ID NO: 1) | PAR of VINC_AQQVSQGLDVLTAK (SEQ ID NO: 9) | 0.842 | 0.764 - 0.902 |
| 9 | VLQGLPR (SEQ ID NO: 1) | PAR of RAB7A_VIILGDSGVGK (SEQ ID NO: 11) | 0.836 | 0.757 - 0.897 |
| 10 | VLQGLPR (SEQ ID NO: 1) | PAR of PDIA6_TGDAIVD.AALS.ALR (SEQ ID NO: 13) | 0.832 | 0.752 - 0.894 |
| 11 | VLQGLPR (SEQ ID NO: 1) | PAR of RAB7A_EAINVEQAFQTIAR (SEQ ID NO: 12) | 0.831 | 0.752 - 0.894 |
| 12 | VLQGLPR (SEQ ID NO: 1) | PAR of RL30_SLESINSR (SEQ ID NO: 15) | 0.831 | 0.752 - 0.893 |
| 13 | VLQGLPR (SEQ ID NO: 1) | PAR of RAN_FNVWDTAGQEK (SEQ ID NO: 17) | 0.830 | 0.750 - 0.892 |
| 14 | VLQGLPR (SEQ ID NO: 1) | PAR of GELS_HVVPNEVVVQR (SEQ ID NO: 18) | 0.828 | 0.749 - 0.891 |
| 15 | VLQGLPR (SEQ ID NO: 1) | PAR of PDIA6_ELSFGR (SEQ ID NO: 14) | 0.827 | 0.747 - 0.890 |
| 16 | VLQGLPR (SEQ ID NO: 1) | PAR of TERA_WALSQSNPSALR (SEQ ID NO: 20) | 0.826 | 0.746 - 0.889 |
| 17 | VLQGLPR (SEQ ID NO: 1) | PAR of RS17_VCEEIAIIPSK (SEQ ID NO: 21) | 0.824 | 0.744 - 0.888 |
| 18 | VLQGLPR (SEQ ID NO: 1) | PAR of COF1_NIILEEGK (SEQ ID NO: 22) | 0.822 | 0.742 - 0.886 |

(continued)

| Num her | HER2 Peptide sequence | Normalization factor | AUROC value | 95% CI |
|---|---|---|---|---|
| 18 | VLQGLPR (SEQ ID NO: 1) | PAR of RL22_AGNLGGGVVTIER (SEQ ID NO: 23) | 0.817 | 0.736 - 0.882 |
| 20 | FVVIQNEDLGPALDSTFYR (SEQ ID NO: 2) | PAR of JAM1_VTFLPTGITFK (SEQ ID NO: 3) | 0.816 | 0.735 - 0.881 |
| 21 | VLQGLPR (SEQ ID NO: 1) | PAR of RS13_LILIESR (SEQ ID NO: 24) | 0.814 | 0.733 - 0.880 |
| 22 | VLQGLPR (SEQ ID NO: 1) | PAR of G3P_GALQNIIPASTGAAK (SEQ ID NO: 25) | 0.813 | 0.732 - 0.878 |
| 23 | VLQGLPR (SEQ ID NO: 1) | PAR of LDHA_VTLTSEEEAR (SEQ ID NO: 26) | 0.813 | 0.731 - 0.878 |
| 24 | VLQGLPR (SEQ ID NO: 1) | Total cell counts | 0.810 | 0.728 - 0.875 |
| 25 | VLQGLPR (SEQ ID NO: 1) | PAR of RL30_LVILANNCPALR (SEQ ID NO: 16) | 0.809 | 0.727 - 0.875 |
| 26 | VLQGLPR (SEQ ID NO: 1) | PAR of GELS_TGAQELLR (SEQ ID NO: 19) | 0.805 | 0.722 - 0.871 |
| 27 | FVVIQNEDLGPALDSTFYR (SEQ ID NO: 2) | PAR of CTND1_GYELLFQPEVVR (SEQ ID NO: 10) | 0.804 | 0.722 - 0.871 |
| 28 | VLQGLPR (SEQ ID NO: 1) | PAR of DESP_AELIVQPELK (SEQ ID NO: 27) | 0.803 | 0.721 - 0.870 |
| 29 | VLQGLPR (SEQ ID NO: 1) | PAR of RL32_AAQLAIR (SEQ ID NO: 28) | 0.803 | 0.720 - 0.870 |
| 30 | VLQGLPR (SEQ ID NO: 1) | Total peptide amount ($\mu$g) | 0.802 | 0.719 - 0.869 |

\* PAR = Peak area ratio

[Table 8]

| Equivocal HER2 cases | | Gold standard (IHC/FISH) | |
|---|---|---|---|
| | | Positive | Negative |
| MRM-MS | Positive | 52 | 7 |
| | Negative | 7 | 54 |

Percent of cases correctly classified : 88.33%

**EXAMPLE 8: Classification of HER2 status using normalized HER2 peptide quantitative analysis values determined by mass spectrometry**

The noramlized value in which the peak area ratio of the three product ions (y6, 683.4199+; y5, 570.3358+; y4, 442.2772+) produced by fragmentation of the HER2 protein peptide (VLQGLPR (SEQ ID NO: 1), 391.7478++) in a collision energy range of 3.1 to 23.1 divided by the peak area ratio of three product ions (y9, 1023.5873+; y8, 876.5189+; y7, 763.4349+) produced by fragmentation of the epithelial cell specific JAM1 peptide (VTFLPTGITFK (SEQ ID NO: 3), 612.3554++) in a collision energy range of 10 to 30 was used to classify the HER2 status of all samples through Mann Whitney U test statistical analysis.

When HER2 scoring is performed based on the normalized HER2 peptide quantitative analysis value, the cutoff value that can be used as a criterion to distinguixh the status was determined as follows: first the normalized HER2 peptide quantitative analysis value was converted to a binomial log (log2), and then an ROC curve was generated that distinguishes adjacent HER2 scores to obtain specificity and sensitivity, and the Youden's J statistic (Ruopp et al. Biom J. 2008 Jun; 50(3):419-430) was used to calculate the value at which the Youden index (J index) becomes the maximum (J index = sensitivity + specificity - 1). As a result, samples with a binomial log value of the normalized HER2 peptide quantitative analysis value of less than -3.4690 was classifed as HER2 0, -3.4690 or more and less than -2.3603 as HER2 1+, and -2.3603 or more and less than -1.9241 as HER2 2+FISH. -1.9241 or more and less than -0.2897 as HER2 2+FISH+, and -0.2897 or more as HER2 3+.

As a result, it was confirmed in the present disclosure that a total of five HER2 status, i.e., HER2 0, 1+, 2+FISH-, 2+FISH+, 3+ can be distinguished with a significance of $p$-value < 0.05 (FIG. 10). Each of 210 samples used herein were separately scored as HER2 0, 1+, 2+FISH, 2+FISH+, or 3+ through IHC and FISH analysis. It was found that the score determined by the conventional methods were in perfect agreement with the status determined by the present method.

In addition, when the present invention is applied in clinical settings, together with a detailed HER2 scoring, it is also important to distinguish between a group that requires Herceptin (traszumab) targeted therapy (HER2-positive breast cancer group) and a group that does not (HER2-negative breast cancer group). It was fouund that the normalized HER2 quantitative analysis values of the present method can distinguish between the HER2 negative group (HER2 0, 1+, 2+FISH-) and the HER2 positive group (HER2 2+FISH+, 3+) with statistical significane of $p$-value < 0.05 (FIG. 11). As shown in FIG. 11, the binomial log value -1.9241 of the noramlized quantitative analysis value of HER2 peptide can be used as a criterion to effectively distinguish HER2-negative (less than -1.9241) and positive (-1.9241 or more) breast cancers.

These results indicates that the present method can be effectively used to determine whether to use Herceptin (traszumab) targeted therapy in clinical settings by perorimg MRM-MS analysis-based quantitative analysis of HER2 peptide and JAM1 peptide followed by calculatation of the cutoff values as shown in FIGS. 10 and 11 to determine HER2 score.

The present method has an advantage over the conventional method in HER2 scoring. The present invention can accurately discriminate between HER2 2+FISH- and HER2 2+FISH+, which cannot be distinguished by the conventional IHC, thereby remarkably reducing the number of HER2 2+ equivocals. This also can lead to the reduction of the cost and time required for FISH by reducing the absolute number of samples that must be tested by FISH, and overall cost and time reduction for HER2 status classification can be achieved (FIG. 12).

**EXAMPLE 9: Comparison of single HER2 peptide peak area ratio and HER2 multi-peptide peak area ratio average value in distinguishing equivocal HER2 group, and comparison of single-marker analysis and multi-marker analysis**

The peak area ratio of the HER2 peptide normalized by the peak area ratio of the JAM1 (VTFLPTGITFK (SEQ ID NO: 3)) peptide as an optimal normalization factor was tested as to distingushing between the HER2 2+FISH- group and the HER2 2+FISH+ group. For this, a total of three peak area ratios of the HER2 peptide were used as follows: (1) the peak area ratio of a single HER2 (VLQGLPR (SEQ ID NO: 1)) peptide, and (2) the average peak area ratio of the two HER2 peptides (VLQGLPR (SEQ ID NO: 1), GLQSLPTHDPSPLQR (SEQ ID NO: 52)) with the highest correlation with a Spearman correlation coefficient ($\rho$) of 0.858, and (3) the average peak area ratio of six HER2 peptides (VLQGLPR (SEQ ID NO: 1), GIWIPDGENVK (SEQ ID NO: 55), LLDIDETEYHADGGK (SEQ ID NO: 53), ELVSEFSR (SEQ ID NO: 54), FVVIQNEDLGPASPLDSTFYR (SEQ ID NO: 2), GLQSLPTHDPSPLQR (SEQ ID NO: 52)). As a result, the HER2 2+FISH- group and the HER2 2+FISH+ group was distinguished as follows: (1) AUC value 0.908 (95% CI, 0.842 - 0.953) when the peak area ratio of a single HER2 (VLQGLPR (SEQ ID NO: 1)) peptide was used having the best result in distinguishing the HER2 2+FISH- and HER2 2+FISH+ groups, and (2) AUC value was 0.858 (95% CI, 0.788 - 0.928) when the average peak area ratio of the two HER2 peptides (VLQGLPR (SEQ ID NO: 1), GLQSLPTHDPSPLQR (SEQ ID NO: 52)) with the highest correlation with a Spearman correlation coefficient ($\rho$) of 0.858 having the next best result in distinguishing the HER2 2+FISH- and HER2 2+FISH+ groups. And, (3) AUC value of 0.827 (95% CI, 0.752 - 0.903) when the average peak area ratio of 6 HER2 peptides (VLQGLPR (SEQ ID NO: 1), GIWIPDGENVK (SEQ ID NO: 55), LLDIDETEYHADGGK (SEQ ID NO: 53), ELVSEFSR (SEQ ID NO: 54), FVVIQNEDLGPASPLDSTFYR (SEQ ID NO: 2), GLQSLPTHDPSPLQR (SEQ ID NO: 52)) was used in distinguishing HER2 2+FISH- and HER2 2+FISH+ group. Taken together, this indicates that the single HER2 peptide (VLQGLPR (SEQ ID NO: 1)) can distinguish between the HER2 2+FISH- and HER2 2+FISH+ groups with AUC value = 0.908, better than using the average value of the peak area ratios of several HER2 peptides (FIG. 15).

In distinguishing between the HER2 2+FISH- group and the HER2 2+FISH+ group using the normalized peak area ratio of the HER2 peptide, the diagnostic performance of the peak area ratio of the HER2 peptide normalized by classical statistical analysis was tested. Among 120 equivocal HER2 samples, a total of 60 randomly selected samples (30 HER2 2+FISH-, 30 HER2 2+FISH+) were used for the training set. Single-marker analysis and multi-marker analyses were performed by logistic regression analysis to determine the best predictive model from each. AUC value was generated by 5-fold cross validation in the training set.

In the single-marker analysis, the peak area ratio of the three product ions (y6, 683.4199+; y5, 570.3358+; y4, 442.2772+) fragmented from the HER2 protein peptide (VLQGLPR (SEQ ID NO: 1), intrinsic mass 391.7478++) in the range of collision energy of 3.1 ~ 23.1 normalized by the peak are ratio of the three product ions (y9, 1023.5873+; y8, 876.5189; y7, 763.4349+) fragmented from the epithelial cell-specific protein JAM1 peptide (VTFLPTGITFK (SEQ ID NO: 3), intrinsic mass 612.3554++) in collision energy in the range of 10 to 30 was found to be the best predictive model. In the training set, it showed that AUC = 0.950, sensitivity = 93.3%, specificity = 93.3%, and in the test set, it showed that AUC = 0.891, sensitivity = 82.8%, specificity = 90.3% (Table 9). In multimarker analysis, the following three marker combination was found to be the best predictive model: (1) the peak area ratio of the three product ions (y6, 683.4199+; y5, 570.3358+; y4, 442.2772) fragmented from HER2 protein peptide (VLQGLPR (SEQ ID NO: 1), intrinsic mass value 391.7478++) in collision energy in the range of 3.1 to 23.1 normalized by the peak area ratio of the three product ions (y9, 1023.5873+; y8, 876.5189+; y7, 763.4349+) fragmented from the epithelial cell-specific protein JAM1 peptide (VTFLPTGITFK (SEQ ID NO: 3), intrinsic mass value 612.3554++) in collision energy in the range of 10 to 30, (2) Normalized peak area ratio of HER2 protein peptide (ELVSEFSR (SEQ ID NO: 54)) with the peak area ratio of RS16 protein peptide (GGGHVAQIYAIR (SEQ ID NO: 51)), and (3) Normalized the peak area ratio of the HER2 protein peptide (VLQGLPR (SEQ ID NO: 1)) with the peak area ratio of the VINC protein peptide (SLGEISALTSK (SEQ ID NO: 6)). In the training set, it showed AUC = 0.969, sensitivity = 93.3%, specificity = 93.3%, and in the test set, it showed AUROC = 0.899, sensitivity = 76.7%, specificity = 96.7% (Table 9).

[Table 9]

| Group | | Single-marker analysis | | Multi-marker analysis | |
|---|---|---|---|---|---|
| | | Training set | Test set | Training set | Test set |
| HER2 2+FISH-vs. HER2 2+FISH+ | AUROC | 0.950 | 0.891 | 0.999 | 0.899 |
| | Sensitivity | 0.933 | 0.828 | 0.933 | 0.767 |
| | Specificity | 0.933 | 0.903 | 0.933 | 0.007 |
| AURDC, area under the receiver operating curve.<br>Single marker: the light-to-heavy peptide peak area ratio (PAR) for the HER2 surrogate peptide (VLQGLPR (SEQ ID NO: 1)), normalized by that for the JAM1 surrogate peptide (VTFLPTGITFK (SEQ ID NO: 3)).<br>Multi-marker: (1) the light-to-heavy peptide PAR for the HER2 surrogate peptide (VLQGLPR (SEQ ID NO: 1)), normalized by that for the JAM1 surrogate peptide (VTFLPTGITFK (SEQ ID NO: 3)), (2) the light-to-heavy peptide PAR for the HER2 surrogate peptide (ELVSEFSR (SEQ ID NO: 54)), normalized by that for the RS16 surrogate peptide (GGGHVAQIYAIR (SEQ ID NO: 51)), and (3) the light-to-heavy peptide PAR for the HER2 surrogate peptide (VLQGLPR (SEQ ID NO: 1)), normalized by that for the VINC surrogate peptide (SLGEISALTSK (SEQ ID NO: 6)). | | | | | |

**EXAMPLE 10: Evaluation of stability and reproducibility according to CPTAC guideline of HER2 (VLQGLPR (SEQ ID NO: 1)) peptide and JAM1 (VTFLPTGITFK (SEQ ID NO: 3)) peptide**

The results are shown in FIGs. 16 and 17, Tables 10 and 11 (stability, reproducibility).

The stability and reproducibility of the three product ions (y6, 683.4199+; y5, 570.3358+; y4, 442.2772+) fragmented from HER2 protein peptide (VLQGLPR (SEQ ID NO: 1), intrinsic mass 391.7478++) in the collision energy in the range of 3.1 to 23.1 and the three product ions (y9, 1023.5873+; y8, 876.5189+; y7, 763.4349+) fragmented from JAM1peptide (VTFLPTGITFK (SEQ ID NO: 3), intrinsic mass 612.3554++) in the collision energy in the range of 10 to 30 were evaluated according to the Clinical Proteomic Tumor Analysis Consortium (CPTAC) guidelines. For the stability evaluation of the HER2 (VLQGLPR (SEQ ID NO: 1)) peptide and JAM1 (VTFLPTGITFK (SEQ ID NO: 3)) peptide according to the CPTAC guideline, the low-quality control (QC) samples and the medium-QC samples each in 6 different storage conditions were analyzed in MRM-MS analysis and then tested whether the target peptides can be stably analyzed in MRM-MS analysis. MRM-MS analysis under 6 conditions: (1) MRM-MS analysis at 4°C immediately after sample preparation (0 hours), (2) MRM-MS analysis after storing the sample at 4°C for 6 hours, (3) MRM-MS analysis after storing the sample at 4°C for 24 hours, (4) MRM-MS analysis by freezing and thawing the sample at 80°C (Batch 2), (5) MRM-MS analysis by freezing and thawing the sample twice at 80°C (Batch 3), (6) MRM-MS analysis after thawing the sample stored at 80° C for 4 weeks (Batch 4).

The variability in the peak area ratio of the HER2 (VLQGLPR (SEQ ID NO: 1)) and JAM1 (VTFLPTGITFK (SEQ ID NO: 3)) peptides of the low-QC sample and the medium-QC sample under six storage conditions was small and all within 7%, which falls within the CPTAC CV % guideline of 20% (A of FIG. 16, Table 10). The variability in the peak area ratio of the HER2 (VLQGLPR (SEQ ID NO: 1)) peptide and the JAM1 (VTFLPTGITFK (SEQ ID NO: 3)) peptide under the remaining five storage conditions at 0 hour in the low-QC sample and the medium-QC sample is 80% to 120% in the recovery which falls within the CPTAC guideline (< ±20% (B of FIG. 16, Table 10).

The reproducibility evaluation of HER2 (VLQGLPR (SEQ ID NO: 1)) peptide and JAM1 (VTFLPTGITFK (SEQ ID NO: 3)) peptide according to the CPTAC guideline is to evaluate the reproducibility of the entire MRM-MS assay workflow. For the HER2 2+FISH- (n=3), and HER2 2+FISH+ (n =3) samples, the tests from sample preps to MRM-MS analysis were performed everyday for 5 days and the variability of the peak area ratio of the HER2 (VLQGLPR (SEQ ID NO: 1)) peptide and the JAM1 (VTFLPTGITFK (SEQ ID NO: 3)) peptide were measured. The variability over 5 days of the HER2 (VLQGLPR (SEQ ID NO: 1)) peptide (FIG. 17, A, Table 11) and JAM1 (VTFLPTGITFK (SEQ ID NO: 3)) peptide (FIG. 17, B, Table 11) peak area ratios in 6 samples was small as CV < 20%. The values of the HER2 (VLQGLPR (SEQ ID NO: 1)) peptide normalized to the JAM1 (VTFLPTGITFK (SEQ ID NO: 3)) peptide also showed small variability with a CV < 20% for 5 days. The normalized value was found to be higher in the HER2 2+FISH+ sample than in the HER2 2+FISH- sample (FIG. 17, C, Table 11), indicating that the present method can accurately determine the expression level of HER2 protein.

[Table 10]

| Target information | | | | Low QC | | | | | | | | | | |
|---|---|---|---|---|---|---|---|---|---|---|---|---|---|---|
| | | | | CV (%) | | | | | | Comparing with Time Zero (%) | | | | |
| Gene Symbol | Uniprot Accession Number | Peptide Sequence | Quantification ion[a] | Batch 1 | | | Batch 2 | Batch 3 | Batch 4 | Batch 1 | | Batch 2 | Batch 3 | Batch 4 |
| | | | | 0 h | 6 h | 24 h | | | | 6 h | 24 h | | | |
| ERBB2 | P04626 | VLQGLPR (SEQ ID NO 1) | 2.y5.1 | 1.41 | 2.04 | 2.12 | 1.91 | 2.38 | 2.08 | 107.60 | 106.67 | 107.33 | 105.02 | 109.25 |
| F11R | Q9Y624 | VTFLPTGITFK (SEQ ID NO: 3) | 2.y7.1 | 4.10 | 2.37 | 4.41 | 1.83 | 3.84 | 3.30 | 106.03 | 101.14 | 93.05 | 93.26 | 103.46 |

| Target information | | | | Medium QC | | | | | | | | | | |
|---|---|---|---|---|---|---|---|---|---|---|---|---|---|---|
| | | | | CV (%) | | | | | | Comparing with Time Zero (%) | | | | |
| Gene Symbol | Uniprot Accession Number | Peptide Sequence | Quantification ion[a] | Batch 1 | | | Batch 2 | Batch 3 | Batch 4 | Batch 1 | | Batch 2 | Batch 3 | Batch 4 |
| | | | | 0 h | 6 h | 24 h | | | | 6 h | 24 h | | | |
| ERBB2 | P04626 | VLQGLPR (SEQ ID NO: 1) | 2.y5.1 | 6.96 | 4.46 | 5.00 | 4.17 | 5.32 | 5.54 | 98.76 | 96.13 | 98.62 | 102.93 | 109.03 |
| F11R | Q9Y624 | VTFLPTGITFK (SEQ ID NO: 3) | 2.y7 1 | 4.28 | 4.37 | 4.50 | 2.52 | 3.01 | 3.77 | 102.25 | 101.34 | 90 85 | 9505 | 96.02 |

QC, quality control; CV, coefficient of variation.

[a]The quantification ion column presents the following quantities separated by periods: precursor ion charge, production ion type, and product ion charge.

[Table 11]

| Target information | | | | | | Inter-day CV (%) | | | | | |
|---|---|---|---|---|---|---|---|---|---|---|---|
| | | | | | | Samples | | | | | |
| Gene Symbol | Uniprot Accession Number | Uniprot ID | Protein Name | Peptide Sequence | Quantification ion[a] | HER2 2+FISH- | | | HER2 2+FISH+ | | |
| | | | | | | 1 | 2 | 3 | 1 | 2 | 3 |
| ERBB2 | P04626 | ERBB2 | Receptor tyrosine-protein kinase erbB-2 | VLQGLPR (SEQ ID NO: 1) | 2.y5.1 | 1.14 | 2.20 | 342 | 4.72 | 1 88 | 1.61 |
| F11R | Q9Y624 | JAM1 | Junctional adhesion molecule A | VTFLPTGITFK (SEQ ID NO: 3) | 2.y7.1 | 3.11 | 11.17 | 5.11 | 12.93 | 6.09 | 0.47 |
| Normalized HER2 level (PAR of VLQGLPR (SEQ ID NO: 1).y51 PAR of VTFLPTGITFK (SEQ ID NO: 3).y7) | | | | | | 3.16 | 10.41 | 6.30 | 8.27 | 5.00 | 1.86 |

QC, quality control; CV, coefficient of variation.

ᵃThe quantification ion column presents the following quantities separated by periods: precursor ion charge, production ion type, and product ion charge.

## EXAMPLE 11: Comparison of time and cost of MRM-MS assay and IHC/FISH method when measuring HER2 status for 30 samples

Here we compared objectively how superior the MRM-MS assay developed in the present invention is in terms of time and cost saved compared to IHC/FISH, a traditional method for evaluation HER2 status. Assuming that the HER2 status of 30 samples was measured, it was confirmed that the MRM-MS assay took 3.4 times less time and 4.6 times less costly than IHC/FISH.

[Table 12]

| Parameter | MRM-MS assay | | IHC | | FISH | |
|---|---|---|---|---|---|---|
| | Time (h) | Cost (USD) | Time (h) | Cost (USD) | Time (h) | Cost (USD) |
| Assay format | Liquid chromatography-mass spectrometry | | Antibody-based immunohistochemical assay | | Gene amplification assay | |
| Specimen type | FFPE tissue | | FFPE tissue | | FFPE tissue | |
| The number of FFPE tissue sections for 30 samples | 80 | | 30 | | 30 | |
| Sample preparation | 15.5 | 1053.54 | 4.15 | 356.13 | 52 | 5,007.51 |
| Consumable items | NA | 78.58 | NA | 23_97 | NA | 35_83 |
| Analytical run (or IHC/FISH imaging) | 6 (12 min / 1 analytical run) | 51.81 (1.73 / 1 analytical run) | 2.5 (5 min / 1 interpretation) | NA | 15 (30 min / 1 interpretation) | NA |
| Total | 21.5 | 1183.93 | 6.65 | 380.10 | 67 | 5,043.34 |

MRM-MS, multiple reaction monitoring-mass spectrometry; Et-OH, ethanol; DTT, dithiothreitol; IAA, iodoacetamide; FA, formic acid; LG, liquid chromatography; Ab, antibody; DAB, 3,3'-Diaminobenzidine; PBS, phosphate-buffered saline; SSC, saline sodium citrate buffer.

The estimated time and costs are based on the analysis of 30 samples. The IHC and FISH procedures are based on the protocol used in actual clinical practice. Because only 15 samples can be performed simultaneously using FISH, the estimated time corresponding to each step is 2-fold that required for 15 samples. The cost units are provided in USD, based on the exchange rate of KRW to USD in February of 2020. The prices for other consumables or reagents that did not significantly impact the total experimental costs were excluded. The labor costs and the operating costs were also excluded, due to variability depending on the type of instrument.

## Claims

1. A method of measuring an expression level of HER2 protein *in vitro* in a breast cancer sample to provide an information for determining HER2 scoring, the method comprising steps of:

    determining an amount of a specific HER2 peptide fragment prepared from the breast cancer sample digested with a protease; and as a normalization factor, determining an amount of the peptide VTFLPTGITFK (SEQ ID NO: 3) from JAM1; and
    normalizing the amount of the specific HER2 peptide with the normalization factor to provide a normalized amount of the HER2 peptide;
    wherein the specific HER2 peptide fragment is VLQGLPR (SEQ ID NO: 1) or FVVIQNEDLGPASPLDSTFYR (SEQ ID NO: 2); and
    wherein the amount of the peptide fragment is determined by a mass spectrometry.

2. The method of claim 1, wherein the sample is FFPE (Formalin-Fixed, Paraffin-Embedded) sample.

3. The method of claim 1, wherein the mass spectrometry includes a tandem mass spectrometry, an ion trap mass spectrometry, a triple quadrupole mass spectrometry, a hybrid ion trap/quadruple mass spectrometry or a time-of-flight mass spectrometry.

4. The method of claim 3, wherein the mode used for the mass spectrometry is SRM (Selected Reaction Monitoring) or MRM (Multiple Reaction Monitoring).

5. The method of any one of claims 1 to 4, wherein the protease is a trypsin.

6. The method of any one of claims 1 to 5, further comprising the step of fractionating the sample digested with a protease before it is used for determining the amount of a specific HER2 peptide fragment and the amount of VTFLPTGITFK (SEQ ID NO: 3) from JAM1.

7. The method of claim 6, wherein the step of fractionation is performed by a liquid chromatography.

8. The method of claim 4, wherein the method is performed in an MRM mode, and the amount of the normalized HER2 peptide is determined by dividing the peak area ratio of three product ions (y6, 683.4199+; y5, 570.3358+; y4, 442.2772+) fragmented from the peptide VLQGLPR (SEQ ID NO: 1) having an intrinsic mass of 391.7478++ by the peak area ratio of three product ions (y9, 1023.5873+; y8, 876.5189+; y7, 763.4349+) fragmented from the JAM1 peptide VTFLPTGITFK (SEQ ID NO: 3) having an intrinsic mass of 612.3554++.

9. The method of claim 4, wherein the method is performed in an MRM mode, and the amount of the normalized HER2 peptide is determined by dividing the peak area ratio of three product ions (y9, 1085.5262+; y8, 998.4942+; b10, 1115.5732+ fragmented from the peptide FVVIQNEDLGPASPLDSTFYR (SEQ ID NO: 2) having the intrinsic mass of 790.0655+++ by the peak area ratio of three product ions (y9, 1023.5873+; y8, 876.5189+; y7, 763.4349+) fragmented from the JAM1 peptide VTFLPTGITFK (SEQ ID NO: 3) having the intrinsic mass of 612.3554++.

10. A method of determining a HER2 score of a breast cancer sample in need thereof using a method according to any one of claims 1 to 9 comprising the step of correlating the normalized amount of HER2 peptide with a HER2 score: 0, 1+, 2+FISH-, 2+FISH+, or 3+.

11. The method of claim 10, wherein the correlation step includes a) as a control, determining a normalized amount of HER2 peptide from breast cancer samples representing each of the HER2 score determined by IHC(Immunohistochemistry) and FISH (Fluorescent In Situ Hybridization) to set a threshold value for each the HER2 score; and b) comparing the normalized HER2 peptide amount from the breast cancer sample in need thereof with the threshold value to assign the score.

12. The method of claim 11, wherein the threshold value is determined using an Youden index (sensitivity + specificity -1) based on the specificity and sensitivity determined by a ROC curve analysis using the normalized amount of HER2 peptide of a)

13. The method of claim 10, wherein the correlation step includes comparing the normalized amount of HER2 peptide with a threshold value determined for each of HER2 score, wherein the HER2 peptide is the peptide of SEQ ID NO: 1, and the amount of the HER2 peptide is normalized by a normalization factor with SEQ ID NO: 3, wherein the threshold value determined for each of HER2 score is: 0 for less than -3.4690, 1+ for -3.4690 or more to less than -2.3603, 2+FISH- for -2.3603 or more to less than -1.9241, 2+FISH+ for -1.9241 or more to less than -0.2897, and 3+ for -0.2897 or more, wherein the normalized amount of the HER2 peptide is a value of log2 of

$$\frac{Peak\ area\ ratio\ of\ HER2\ peptide\ (SEQ\ ID\ NO{:}1)}{Peak\ area\ ratio\ of\ JAM1\ peptide\ (VTFLPTGITFK\ (SEQ\ ID\ NO{:}3))}$$

in which the peak area ratio of the product ion of the HER2 peptide is divided by the peak area ratio of the product ion of the normalization factor; or
wherein the correlation step includes comparing the normalized amount of HER2 peptide with a threshold value determined for each of HER2 score, wherein the HER2 peptide is the peptide of SEQ ID NO: 2, and the amount of the HER2 peptide is normalized by a normalization factor with SEQ ID NO: 3, wherein the threshold value determined for each of HER2 score is: 0 for less than -1.8208, 1+ for -1.8208 or more to less than -1.2673, 2+FISH- for -1.2673 or more to less than -0.5299, 2+FISH+ for -0.5299 or more to less than 0.9543, and 3+ for -0.9543 or more, wherein the normalized amount of the HER2 peptide is a value of log2 of

$$\frac{Peak\ area\ ratio\ of\ HER2\ peptide\ (SEQ\ ID\ NO:2)}{Peak\ area\ ratio\ of\ JAM1\ peptide\ (VTFLPTGITFK\ (SEQ\ ID\ NO:3))}$$

in which the peak area ratio of the product ion of the HER2 peptide is divided by the peak area ratio of the product ion of the normalization factor.

**Patentansprüche**

1. Verfahren zum Messen eines Expressionsniveaus von HER2-Protein in vitro in einer Brustkrebsprobe, um Informationen bereitzustellen, um eine HER2-Auswertung zu bestimmen, wobei das Verfahren die folgenden Schritte umfasst:

    Bestimmen einer Menge eines spezifischen HER2-Peptidfragments, das aus der mit einer Protease digerierten Brustkrebsprobe erstellt wird; und Bestimmen als einen Normierungsfaktor einer Menge des Peptids VTFLPTGITFK (SEQ ID NO: 3) von JAM1; und
    Normieren der Menge des speifischen HER2-Peptids mit dem Normierungsfaktor, um eine normierte Menge des HER2-Peptids bereitzustellen;
    wobei das spezifische HER2-Peptidfragment VLQGLPR (SEQ ID NO: 1) oder FVVIQNEDLGPASPLDSTFYR (SEQ ID NO: 2) ist; und
    wobei die Menge des Peptidfragments durch Massenspektrometrie bestimmt wird.

2. Verfahren nach Anspruch 1, wobei die Probe eine FFPE-Probe (formalinfixierte in Paraffin eingebettete Probe) ist.

3. Verfahren nach Anspruch 1, wobei die Massenspektrometrie eine Tandemmassenspektrometrie, eine Ionenfallenmassenspektrometrie, eine Dreifachquadrupolmassenspektrometrie, eine Hybridionenfallen-/Quadrupolmassenspektrometrie oder eine Flugzeitmassenspektrometrie enthält.

4. Verfahren nach Anspruch 3, wobei die Betriebsart, die für die Massenspektrometrie verwendet wird, SRM, (ausgewählte Reaktionsüberwachung) oder MRM (Mehrfachreaktionsüberwachung) ist.

5. Verfahren nach einem der Ansprüche 1 bis 4, wobei die Protease ein Trypsin ist.

6. Verfahren nach einem der Ansprüche 1 bis 5, das ferner den Schritt umfasst, die mit einer Protease digerierte Probe zu fraktionieren, bevor sie verwendet wird, um die Menge eines spezifischen HER2-Peptidfragments und die Menge von VTFLPTGITFK (SEQ ID NO: 3) von JAM1 zu bestimmen.

7. Verfahren nach Anspruch 6, wobei der Schritt der Fraktionierung durch eine Flüssigchromatographie ausgeführt wird.

8. Verfahren nach Anspruch 4, wobei das Verfahren in einer MRM-Betriebsart ausgeführt wird und die Menge des normierten HER2-Peptids durch Teilen des Spitzenflächenverhältnisses von drei Produktionen (y6, 683.4199+; y5, 570.3358+; y4, 442.2772+), die von dem Peptid VLQGLPR (SEQ ID NO: 1) fragmentiert wurden, das eine intrinsische Masse von 391.7478++ besitzt, durch das Spitzenflächenverhältnis von drei Produktionen (y9, 1023.5873+; y8, 876.5189+; y7, 763.4349+), die von dem JAM1-Peptid VTFLPTGITFK (SEQ ID NO: 3) fragmentiert wurden, das eine intrinsische Masse von 612.3555++ besitzt, bestimmt wird.

9. Verfahren nach Anspruch 4, wobei das Verfahren in einer MRM-Betriebsart ausgeführt wird und die Menge des normierten HER2-Peptids durch Teilen des Spitzenflächenverhältnisses von drei Produktionen (y9, 1085.5262+; y8, 998.4942+; b10, 1115.5732+, die von dem Peptid FVVIQNEDLGPASPLDSTFYR (SEQ ID NO: 2) fragmentiert wurden, das eine intrinsische Masse von 790.0655+++ besitzt, durch das Spitzenflächenverhältnis von drei Produktionen (y9, 1023.5873+; y8, 876.5189+; y7, 763.4349+), die von dem JAM1-Peptid VTFLPTGITFK (SEQ ID NO: 3) fragmentiert wurden, das eine intrinsische Masse von 612.3555++ besitzt, bestimmt wird.

**10.** Verfahren zum Bestimmen einer HER2-Einstufung einer Brustkrebsprobe, die eine solche benötigt, unter Verwendung eines Verfahrens nach einem der Ansprüche 1 bis 9, das den Schritt umfasst, die normierte Menge des HER2-Peptids mit einer HER2-Einstufung zu korrelieren: 0, 1+, 2+FISH-, 2+FISH+ oder 3+.

**11.** Verfahren nach Anspruch 10, wobei der Korrelationsschritt enthält: a) als eine Kontrolle Bestimmen einer normierten Menge des HER2-Peptids aus Brustkrebsproben, die jeweils die HER2-Einstufung repräsentieren, die durch IHC (Immunhistochemie) und FISH (Fluoreszent-in-situ-Hybridisierung) bestimmt wurde, um einen Schwellenwert für jede der HER2-Einstufungen einzustellen; und b) Vergleichen der normierte HER2-Peptidmenge von der Brustkrebsprobe, die eine solche benötigt, mit dem Schwellenwert, um die Einstufung zuzuweisen.

**12.** Verfahren nach Anspruch 11, wobei der Schwellenwert unter Verwendung eines Youden-Index (Empfindlichkeit + Spezifizität -1) anhand der Spezifizität und der Empfindlichkeit, die durch eine ROC-Kurvenanalyse unter Verwendung der normierten Menge von HER2-Peptid von a) bestimmt wurden, bestimmt wird.

**13.** Verfahren nach Anspruch 10, wobei der Korrelationsschritt enthält, die normierte Menge des HER2-Peptids mit einem Schwellenwert zu vergleichen, der für jede HER2-Einstufung bestimmt wird, wobei das HER2-Peptid ein Peptid von SEQ ID NO: 1 ist und die Menge des HER2-Peptids durch einen Normierungsfaktor mit SEQ ID NO: 3 normiert ist, wobei der Schwellenwert, der für jede HER2-Einstufung bestimmt wird, ist: 0 für weniger als -3.4590, 1+ für -3.4690 oder mehr bis weniger als -2.3603, 2+FISH für -2.3603 oder mehr bis weniger als -1.9241, 2+FISH+ für -1.9241 oder mehr bis weniger als -0.2897 und 3+ für -0.2897 oder mehr, wobei die normierte Menge des HER2-Peptids ein Wert von Log2 von

$$\frac{Spitzenflächenverhältnis\ von\ HER2-Peptid\ (SEQ\ ID\ NO:1)}{Spitzenflächenverhältnis\ von\ JAM1-Peptid\ (VTFLPTGITFK\ (SEQ\ ID\ NO:3))}$$

ist, wobei das Spitzenflächenverhältnis des Produktions des HER2-Peptids durch das Spitzenflächenverhältnis des Produktions des Normierungsfaktors geteilt wird; oder

wobei der Korrelationsschritt enthält, die normierte Menge des HER2-Peptids mit einem Schwellenwert zu vergleichen, der für jede HER2-Einstufung bestimmt wird, wobei das HER2-Peptid das Peptid von SEQ ID NO: 2 ist und die Menge des HER2-Peptids durch einen Normierungsfaktor mit SEQ ID NO: 3 normiert ist, wobei der Schwellenwert, der für jede HER2-Einstufung bestimmt wird, ist: 0 für weniger als -1.8208, 1+ für -1.8208 oder mehr bis weniger als -1.2673, 2+FISH- für -1.2673 oder mehr bis weniger als -0.5299, 2+FISH+ für -0.5299 oder mehr bis weniger als 0.9543 und 3+ für -0.9543 oder mehr, wobei die normierte Menge des HER2-Peptids ein Wert von Log2 von

$$\frac{Spitzenflächenverhältnis\ von\ HER2-Peptid\ (SEQ\ ID\ NO:2)}{Spitzenflächenverhältnis\ von\ JAM1-Peptid\ (VTFLPTGITFK\ (SEQ\ ID\ NO:3))}$$

ist, wobei das Spitzenflächenverhältnis des Produktions des HER2-Peptids durch das Spitzenflächenverhältnis des Produktions des Normierungsfaktors geteilt wird.

## Revendications

**1.** Procédé de mesure d'un niveau d'expression de la protéine HER2 *in vitro* dans un échantillon de cancer du sein pour fournir une information destinée à déterminer un score HER2, le procédé comportant les étapes consistant à :

déterminer une quantité d'un fragment de peptide HER2 spécifique, préparé à partir de l'échantillon de cancer du sein digéré par une protéase ; et en tant que facteur de normalisation, déterminer une quantité du peptide VTFLPTGITFK (SÉQ ID N° : 3) à partir de JAM1 ; et

normaliser la quantité du peptide HER2 spécifique avec le facteur de normalisation pour fournir une quantité normalisée du peptide HER2 ;
dans lequel le fragment de peptide HER2 spécifique est VLQGLPR (SÉQ ID N° 1) ou FVVIQNEDLGPASPLDSTFYR (SÉQ ID N° 2) ; et

dans lequel la quantité du fragment de peptide est déterminée par une spectrométrie de masse.

2. Procédé selon la revendication 1, dans lequel l'échantillon est un échantillon FFPE (fixé au formol et inclus en paraffine).

3. Procédé selon la revendication 1, dans lequel la spectrométrie de masse inclut une spectrométrie de masse en tandem, une spectrométrie de masse à piège ionique, une spectrométrie de masse à triple quadripôle, une spectrométrie de masse hybride à piège ionique/quadripôle ou une spectrométrie de masse à temps de vol.

4. Procédé selon la revendication 3, dans lequel le mode utilisé pour la spectrométrie de masse est SRM (surveillance de réactions sélectionnées) ou MRM (surveillance de réactions multiples).

5. Procédé selon l'une quelconque des revendications 1 à 4, dans lequel la protéase est une trypsine.

6. Procédé selon l'une quelconque des revendications 1 à 5, comportant en outre l'étape consistant à fractionner l'échantillon digéré par une protéase avant de l'utiliser pour déterminer la quantité d'un fragment de peptide HER2 spécifique et la quantité de VTFLPTGITFK (SÉQ ID N° 3) à partir de JAM1.

7. Procédé selon la revendication 6, dans lequel l'étape de fractionnement est réalisée par une chromatographie en phase liquide.

8. Procédé selon la revendication 4, dans lequel le procédé est mis en oeuvre dans un mode MRM, et la quantité du peptide HER2 normalisé est déterminée en divisant le rapport des aires des pics de trois ions de produit (y6, 683.4199+; y5, 570.3358+; y4, 442.2772+) fragmentés à partir du peptide VLQGLPR (SÉQ ID N° 1) ayant une masse intrinsèque de 391.7478++ par le rapport des aires des pics de trois ions de produit (y9, 1023.5873+; y8, 876.5189+; y7, 763.4349+) fragmentés à partir du peptide JAM1 VTFLPTGITFK (SÉQ ID N° 3) ayant une masse intrinsèque de 612.3554++.

9. Procédé selon la revendication 4, dans lequel le procédé est mis en oeuvre dans un mode MRM, et la quantité du peptide HER2 normalisé est déterminée en divisant le rapport des aires des pics de trois ions de produit (y9, 1085.5262+; y8, 998.4942+; b10, 1115.5732+) fragmentés à partir du peptide FVVIQNEDLGPASPLDSTFYR (SÉQ ID N° 2) ayant la masse intrinsèque de 790.0655+++ par le rapport des aires des pics de trois ions de produit (y9, 1023.5873+; y8, 876.5189+; y7, 763.4349+) fragmentés à partir du peptide JAM1 VTFLPTGITFK (SÉQ ID N° 3) ayant la masse intrinsèque de 612.3554++.

10. Procédé pour déterminer un score HER2 d'un échantillon de cancer du sein nécessitant un tel score en utilisant un procédé selon l'une quelconque des revendications 1 à 9 comportant l'étape consistant à mettre en corrélation la quantité normalisée de peptide HER2 avec un score HER2 : 0, 1+, 2+FISH-, 2+FISH+ ou 3+.

11. Procédé selon la revendication 10, dans lequel l'étape de corrélation inclut de a) déterminer, en tant que témoin, une quantité normalisée de peptide HER2 à partir d'échantillons de cancer du sein représentant chaque score HER2 déterminé par IHC (immunohistochimie) et FISH (hybridation in situ en fluorescence) pour fixer une valeur de seuil pour chaque score HER2 ; et b) comparer la quantité de peptide HER2 normalisée provenant de l'échantillon de cancer du sein qui en a besoin avec la valeur de seuil afin d'attribuer le score.

12. Procédé selon la revendication 11, dans lequel la valeur de seuil est déterminée en utilisant un indice de Youden (sensibilité + spécificité -1) basé sur la spécificité et la sensibilité déterminées par une analyse de courbe ROC en utilisant la quantité normalisée de peptide HER2 de a).

13. Procédé selon la revendication 10, dans lequel l'étape de corrélation inclut la comparaison de la quantité normalisée de peptide HER2 avec une valeur de seuil déterminée pour chaque score HER2, dans lequel le peptide HER2 est le peptide de la SÉQ ID N° 1, et la quantité du peptide HER2 est normalisée par un facteur de normalisation avec la SÉQ ID N° 3, dans lequel la valeur de seuil déterminée pour chaque score HER2 est : 0 pour moins de -3.4690, 1+ pour -3.4690 ou plus jusqu'à moins de -2.3603, 2+FISH- pour -2.3603 ou plus jusqu'à moins de -1.9241, 2+FISH+ pour -1.9241 ou plus jusqu'à moins de -0.2897, et 3+ pour -0.2897 ou plus, dans lequel la quantité normalisée du peptide HER2 est une valeur log2 de :

$$\frac{\textit{Rapport des aires des pics du peptide HER2 (SÉQ ID N° 1)}}{\textit{Rapport des aires des pics du peptide JAM1 (VTFLPTGITFK (SÉQ ID N° 3))}}$$

dans laquelle le rapport des aires des pics de l'ion de produit du peptide HER2 est divisé par le rapport des aires des pics de l'ion de produit du facteur de normalisation ; ou

dans lequel l'étape de corrélation inclut la comparaison de la quantité normalisée de peptide HER2 avec une valeur de seuil déterminée pour chaque score HER2, dans lequel le peptide HER2 est le peptide de la SÉQ ID N° 2, et la quantité du peptide HER2 est normalisée par un facteur de normalisation avec la SÉQ ID N° 3, dans lequel la valeur de seuil déterminée pour chaque score HER2 est : 0 pour moins de -1.8208, 1+ pour -1.8208 ou plus jusqu'à moins de -1.2673, 2+FISH- pour -1.2673 ou plus jusqu'à moins de -0.5299, 2+FISH+ pour -0.5299 ou plus jusqu'à moins de 0.9543, et 3+ pour -0.9543 ou plus, dans lequel la quantité normalisée du peptide HER2 est une valeur log2 de

$$\frac{\textit{Rapport des aires des pics du peptide HER2 (SÉQ ID N° 2)}}{\textit{Rapport des aires des pics du peptide JAM1 (VTFLPTGITFK (SÉQ ID N° 3))}}$$

dans laquelle le rapport des aires des pics de l'ion de produit du peptide HER2 est divisé par le rapport des aires des pics de l'ion de produit du facteur de normalisation.

FIG. 1

## FIG. 2

FIG. 3

**FIG. 4**

FIG. 5

FIG. 6

# FIG. 7A

## FIG. 7B

FIG. 8

HER2 2+/FISH– vs. HER2 2+/FISH+

FIG. 9

## FIG. 10

FIG. 11

Clinical application of MRM-MS based standard measurement method
for targeted therapy for HER2-positive breast cancer

FIG. 12

# FIG. 13

**A**

HER2.VLQGLPR.y5

m/z = 391.7 / 570.3
m/z = 396.7 / 580.3

Intensity (10^3)

Retention time (min)

**B**

JAM1.VTFLPTGITFK.y7

m/z = 612.3 / 763.4
m/z = 616.3 / 771.4

Intensity

Retention time (min)

**C**

| Protein<br>Peptide Sequence | HER2<br>VLQGLPR | JAM1<br>VTFLPTGITFK |
|---|---|---|
| **Purified peptide<br>(light, unlabeled form)** | | |
| Mean area | 337907.33 | 43815.00 |
| SD | 33647.21 | 283.93 |
| CV (%) | 9.96 | 0.65 |
| **Unpurified peptide<br>(heavy, labeled form)** | | |
| Mean area | 149656.67 | 19232.00 |
| SD | 14625.73 | 555.88 |
| CV(%) | 9.77 | 2.89 |
| Peak area ratio<br>(H/L) | 0.44 | 0.44 |
| SD | 0.00 | 0.01 |
| CV (%) | 0.25 | 2.66 |
| Purity (%) | 44.30 | 43.89 |

* P < 0.05, NA not available

46

EP 3 936 870 B1

FIG. 14

## FIG. 15

HER2 2+/FISH– vs. HER2 2+/FISH+

Y-axis: Sensitivity %
X-axis: 100 % - Specificity %

Legend:
HER2 peptide / JAM1 peptide
(VLQGLPR)    (VTFLPTGITFK)
: AUROC 0.908 (95% CI, 0.842 – 0.953)

Average of VLQ. and GLQ. of HER2 with high correlation / JAM1 peptide
: AUROC 0.858 (95% CI, 0.788 – 0.928)

Average of six peptides of HER2 / JAM1 peptide
: AUROC 0.827 (95% CI, 0.752 – 0.903)

**FIG. 16**

## FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130302328 A

- KR 20180036653

**Non-patent literature cited in the description**

- **C. STEINER et al.** Quantification of HER2 by Targeted Mass Spectrometry in Formalin-Fixed Paraffin-Embedded (FFPE) Breast Cancer Tissues. *MOLECULAR & CELLULAR PROTEOMICS, US,* 06 July 2015, vol. 14 (10), 2786-2799

- **RUOPP et al.** *Biom J.,* June 2008, vol. 50 (3), 419-430